# FASCICULE DE BREVET EUROPEEN

(11) **EP 3 565 902 B1**
(45) Date de publication et mention de la délivrance du brevet: **04.09.2024**
(21) Numéro de dépôt: 18703044.0
(22) Date de dépôt: 08.01.2018
(51) Int. Cl.: C12Q 1/6806, C12Q 1/6886

(54) **PROCEDE DE DETECTION ET QUANTIFICATION D'ADN CIRCULANT ET UTILISATIONS**
VERFAHREN ZUM NACHWEIS UND ZUR QUANTIFIZIERUNG VON ZIRKULIERENDER DNA UND VERWENDUNGEN
METHOD FOR DETECTING AND QUANTIFYING CIRCULATING DNA AND USES

(30) Priorité: 06.01.2017 FR 1750146
(43) Date de publication de la demande: 13.11.2019
(73) Titulaire: ID Solutions Oncology, 13016 Marseille (FR)
(72) Inventeur: FINA, Frédéric, 13850 Greasque (FR); POURQUIER, Philippe, 34070 Montpellier (FR); GREWIS, Lise, 34980 Combaillaux (FR)
(74) Mandataire: Regimbeau
(86) Numéro de dépôt international: PCT/FR2018/050038
(87) Numéro de publication internationale: WO 2018/127674

(56) Documents cités:
- WO-A2-2014/194113
- MARYAM EINI ET AL: "Chimeric External Control to Quantify Cell Free DNA in Plasma Samples by Real Time PCR", AVICENNA JOURNAL OF MEDICAL BIOTECHNOLOGY, 5 March 2016 (2016-03-05), Iran, pages 84, XP055407344, Retrieved from the Internet <URL:https://www.ncbi.nlm.nih.gov/pmc/articles/PMC4842246/pdf/AJMB-8-84.pdf> [retrieved on 20170918]
- DEVONSHIRE ALISON S ET AL: "Towards standardisation of cell-free DNA measurement in plasma: controls for extraction efficiency, fragment size bias and quantification", ANALYTICAL AND BIOANALYTICAL CHEMISTRY, SPRINGER, DE, vol. 406, no. 26, 24 May 2014 (2014-05-24), pages 6499 - 6512, XP035401224, ISSN: 1618-2642, [retrieved on 20140524], DOI: 10.1007/S00216-014-7835-3
- GORMALLY EMMANUELLE ET AL: "Circulating free DNA in plasma or serum as biomarker of carcinogenesis: Practical aspects and biological significance", MUTATION RESEARCH. REVIEWS IN MUTATION RESEARCH, ELSEVIER, AMSTERDAM, NL, vol. 635, no. 2-3, 25 January 2007 (2007-01-25), pages 105 - 117, XP002599420, ISSN: 1383-5742, [retrieved on 20070125], DOI: 10.1016/J.MRREV.2006.11.002
- SUTHEE RAPISUWON ET AL: "Circulating biomarkers to monitor cancer progression and treatment", COMPUTATIONAL AND STRUCTURAL BIOTECHNOLOGY JOURNAL, vol. 14, 1 June 2016 (2016-06-01), Sweden, pages 211 - 222, XP055407446, ISSN: 2001-0370, DOI: 10.1016/j.csbj.2016.05.004
- CLEMENS HUFNAGL ET AL: "A modified Phenol-chloroform extraction method for isolating circulating cell free DNA of tumor patients", JOURNAL OF NUCLEIC ACIDS INVESTIGATION, vol. 4, no. 1, 11 March 2013 (2013-03-11), pages 1 - 3, XP055407539, ISSN: 2035-6005, DOI: 10.4081/jnai.2013.e1

## Description

### DOMAINE DE L'INVENTION

Le domaine de l'invention concerne un procédé de détection et de quantification d'ADN libre de toutes cellules (autrement nommé de façon générale `ADN circulant' ou `ADN libre circulant ADNcf') et leur utilisation notamment à des fins de diagnostic, pronostic ou suivi de l'état physiologique d'un sujet d'intérêt donné.

### ETAT DE L'ART

On sait depuis de nombreuses années que le plasma sanguin contient de petites quantités d'ADN libre circulant, de l'ordre d'une dizaine de nanogrammes par millilitre, cette concentration pouvant s'élever significativement, jusqu'à 0,5 microgramme par millilitre, chez les patients atteints de cancer. Plusieurs études ont montré que l'on pouvait, à partir de cet ADN circulant, détecter des modifications génétiques spécifiques des processus cancéreux comme des mutations et des altérations des microsatellites (cancers pulmonaires, colorectaux, de la tête et du cou). La prévalence de ces altérations génétiques de l'ADN circulant varie cependant selon les maladies et les gènes étudiés (Pierre Hainaut, m/s n°3, vol.16, mars 2000).

Si, actuellement les concentrations en ADN circulants ne sont pas ou peu utilisées comme outil diagnostique ou pronostique, c'est par manque d'une standardisation des méthodes d'extraction et de dosage qui permettrait d'établir ces seuils standardisés. Des "bonnes pratiques" pré-analytiques spécifiques aux ADN circulants ont été publiées. Ils recommandent notamment de travailler avec du plasma collecté dans des tubes (EDTA, Streck, Roche), de centrifuger le tube de sang au plus tard 6 h après le prélèvement (jusqu'à 24h selon les centres d'analyse), de conserver le plasma entre - 20°C (conservation < 1 mois) et - 80°C(conservation > 1 mois), de conserver les ADN circulants extraits à - 20 °C (en tube vissé) et d'éviter les cycles de décongélation-recongélation répétés.

Cependant, les considérations pré-analytiques qui influencent le résultat analytique doivent prendre en considération d'autres facteurs tels que le volume de plasma à extraire, directement corrélé à la quantité d'ADN extrait, le rendement de la technique d'extraction pour un volume donné de liquide, la reproductibilité de l'extraction, la concentration finale de l'ADN et la standardisation inter-essais du rendement/concentration de l'éluat obtenu.

En raison de la relative facilité d'accès et la nature de l'échantillon, l'utilisation de l'ADN libre de toute cellule (autrement nommé ADN circulant) est depuis maintenant quelques années pressentie pour le suivi de la progression ou de la réponse des patients aux traitements anticancéreux (Aung KL et al., Hugo J 2010) ou autres. Le terme de «biopsie liquide» a émergé pour décrire la surveillance de l'évolution génétique de la tumeur à partir du sang (Fatouros IG et al.,ClinBiochem 2010).

Les concentrations en ADN circulants reflètent en effet des processus pathologiques mais également physiologiques (Fatouros IG, et al. ClinBiochem 2010). L'ADN extracellulaire circulant peut-être détecté chez les témoins sains (Fatouros IG, et al. ClinBiochem 2010) et atteints d'affections malignes (Oxnard GR et al., Clin Cancer Res 2014 ; Lipson EJ et al., J Immunother Cancer 2014 ; Reinert T et al., Gut 2016 ; ) ou non malignes (Steinman CR. J Clin Invest 1984 ; Galeazzi M, et al. AutoimmunRev 2003). En outre, les traumatismes (Laktionov PP, et al., Nucleosides Nucléotides NucleicAcids 2004), procédures thérapeutiques (Davis GL, Jr., Davis JS., ArthritisRheum 1973) et la gestation (Lo YM et al, 1997) peuvent aussi entraîner la libération d'ADN libre dans la circulation. De nombreux facteurs peuvent donc influencer le niveau de l'ADN circulant dans le plasma. Le suivi de la réponse aux traitements par prise de sang régulière présente de réels avantages.

Ainsi, si la quantité d'ADN circulant par ml de liquide peut être considérée comme un biomarqueur d'une réponse clinique, la standardisation de la quantité d'ADN circulant extrait d'une extraction à l'autre est primordiale.

En plus de la standardisation de la quantité d'ADN extrait, conserver le profil de taille des échantillons d'ADN circulant revêt une importance qui peut s'avérer capitale pour la validation biologique des résultats. En effet, la taille des fragments d'ADN circulants, chez les témoins sains provient essentiellement des cellules apoptotiques. Les longueurs des fragments d'ADN circulant sont généralement de 185 à 200 bp (Giacona MB et al., Pancreas 1998). Cet ADN circulant uniformément tronqué est produit par clivage enzymatique au cours de l'apoptose (Wyllie AH, Nature 1980). En ce qui concerne la taille de l'ADN circulant provenant des cellules malignes, elle varie beaucoup, car outre l'apoptose, la nécrose, l'autophagie sont responsables de la mort des cellules cancéreuses (Jin Z and El-Deiry WS, Cancer BiolTher 2005). La taille des fragments d'ADN circulant est donc différente entre les individus sains et pathologiques.

Il est important que les techniques d'extraction d'ADN circulant conservent le profil en taille des ADN circulants, en particulier les fragments de petites tailles qui peuvent être éliminées si la proportion du mélange des composants chimiques de l'étape de fixation sur la colonne de silice, varie. La composition du liquide peut également modifier cette proportion. Il est donc primordial de pouvoir vérifier et normaliser l'efficacité de l'extraction des fragments de petite taille, en particulier autour de 100 paires de bases.

D'autres paramètres sont également à prendre en compte, notamment le rendement d'extraction d'ADN (quantité totale), le volume d'élution et la concentration de ces ADN circulants.

En effet, la quantité d'ADN circulant est directement proportionnelle à la quantité de plasma extrait, Il est donc important d'extraire un maximum de plasma. Le volume total à extraire incluant plasma, protéases et solutions de fixation dépasse en moyenne les 10 ml (pour 4 ml de plasma) et doit être élué sur des colonnes de silice ne dépassant pas 1 à 2 cm de hauteur et 1 cm de diamètre. Il faut noter que plus le diamètre de la colonne est petit, plus le volume d'élution sera réduit et donc l'extrait d'ADN circulant concentré.

Par ailleurs, le nombre d'analyse à réaliser sur ce type d'échantillon peut être très important ; il est ainsi primordial d'obtenir des volumes d'élutions qui permettront de réaliser de nombreuses analyses avec des concentrations d'ADN circulant suffisante. Les deux sont antinomiques.

Enfin, l'obtention d'une solution concentrée est primordiale car la sensibilité analytique est directement dépendante de la quantité d'ADN circulant analysée.

Le volume de liquide à extraire, la concentration et le volume d'élution sont interdépendants.

A la connaissance de la Demanderesse, il n'existe pas actuellement de kits et dispositifs d'extraction, d'amplification et de quantification d'ADN, en particulier d'ADN libre de toutes cellules, répondant à ces différentes problématiques.

Par ailleurs, depuis les travaux de P. Mandel et P. Métais en 1948, il est clairement établi que le sang véhicule une petite quantité d'ADN libre circulant provenant de la libération de matériel génétique par les tissus. Cet ADN libre circulant (ADNcf) est sous la forme d'ADN double-brin d'une taille moyenne de 150-180 pb correspondant à l'enroulement de l'ADN autour du nucléosome, les tailles supérieures correspondent à des multiples de 150-180pb. Sa durée de vie est de moins de deux heures, avant qu'il ne soit filtré et éliminé de la circulation sanguine par la rate, le foie et les reins. Toutes les études s'accordent à dire que l'ADNcf est détecté en moindre quantité chez les individus sains et que son augmentation est liée à différentes situations cliniques telles que notamment AVC, infarctus myocardique, exercice musculaire intensif, insuffisance rénale aiguë, cytolyse hépatique, traumatismes, chirurgie, rejet de greffes et cancer.

L'origine de l'ADNcf n'est pas encore parfaitement élucidée mais serait liée à 3 phénomènes : l'apoptose, la nécrose et dans une moindre mesure la sécrétion active. Chez les individus sains, l'ADN circulant provient essentiellement des cellules en apoptose (Giacona, M. B. et al., Pancréas, 1998). Les longueurs des fragments d'ADN sont dans ce cas généralement de 185 à 200 bp (Wyllie, A. H. et al., Int Rev Cytol, 1980). En revanche, dans les tissus cancéreux, la taille de l'ADN circulant varie beaucoup, car outre l'apoptose, la nécrose et l'autophagie sont responsables de la mort des cellules cancéreuses (Jin, Z. et al., Cancer Biol Ther, 2005). Les fragments peuvent atteindre 450-500 pb correspondant à l'enroulement de l'ADN autour de 3 nucléosomes. Au-delà de ces tailles, des fragments plus grands encore, qui sont le plus souvent considérés comme résultant d'une contamination par de l'ADN leucocytaire, peuvent également être présents. Toutefois l'origine de ces fragments de haut poids n'est pas clairement élucidée. Il est possible de distinguer les populations d'ADN circulant en mesurant la taille des fragments longs et des fragments courts. Il s'agit d'approches NGS (Next Génération Sequencing) utilisant des processus de bio-informatique spécifiques permettant la mesure de la taille des ADNcf. Jiang P et Lo YM montrent un intérêt pour de nombreuses applications, particulièrement dans le domaine de la cancérologie et de la transplantation (Trends Genet. 2016). Malgré tout, il est à ce jour impossible, en routine clinique, de s'assurer de l'origine somatique (tumeur, transplant,...) et du processus de libération de l'ADN circulant (apoptose, nécrose, ...).

Un des écueils majeurs de l'analyse des ADNcf concerne les processus pré analytiques (traitement des tubes de sang et extraction des ADN). Les variations de concentrations en ADNcf peuvent être artéfactuellement produites par la libération d'ADN leucocytaires avant la première centrifugation des tubes, ou encore en absence d'une seconde centrifugation à minimum 4000g. De plus, dans l'optique d'une utilisation des concentrations en ADNcf exprimées par ml/plasma pour suivre une réponse aux traitements, il est important de s'assurer de la robustesse de la technique d'extraction, mais surtout proposer une méthode permettant de normaliser les extractions entre elles et au cours du temps.

Eini et al. (Avicenna J Med Biotech 2016 ; 8(2) :84-90) décrit une méthode d'extraction *'in house'* et une méthode d'amplification de l'ADN circulant dans un échantillon de plasma de sujet sain, mettant en oeuvre un fragment d'ADN exogène de 167 nucléotides et le calcul d'un ratio d'intégrité de l'ADN extrait pour une évaluation qualitative de l'étape d'extraction.

WO2014/194113 (Schutz et al.) décrit une méthode de quantification d'ADN acellulaire chez un sujet greffé à partir d'un échantillon de plasma enrichi avec un ADN de 320 pb d'origine non humaine, pour l'évaluation qualitative du rendement d'extraction.

Mais aucun de ces documents ne décrit ni ne suggère le procédé objet des revendications 1 à 6 visant à co-amplifier et quantifier un ADN circulant extrait à partir d'un échantillon biologique d'un sujet atteint ou développant une condition clinique pathologique telle que définie selon l'invention, et un fragment d'ADN exogène de 50-200 paires de bases, afin de détecter selon l'invention des variations quantitatives ADN circulant extrait, à finalité de diagnostic ou pronostic.

Les inventeurs ont justement mis au point un procédé de détection et de quantification d'ADN libres de toutes cellules (ADN circulants), comprenant une étape d'extraction dans laquelle sont ajoutés des calibrants autrement nommés standards (fragments d'ADN exogène) permettant de standardiser les variations d'ADN circulant chez les patients, et de vérifier éventuellement par ailleurs la reproductibilité d'extraction des ADN circulants de petite taille.

Par 'calibrants' ou 'standards' selon l'invention, on entend des contrôles internes du procédé de détection et quantification de l'ADN circulant extrait, lesdits calibrants ou standards étant constitués par des fragments d'ADN exogène définis ci-après. On parlera également de fragment ICE calibré ou fragment ICE dans le reste de la description.

Par `fragment ICE calibré', on entend selon l'invention un fragment d'ADN exogène dont la concentration est étalonnée par ddPCR ; l'utilisation de ce fragment ICE calibré permet de normaliser la concentration d'ADN génomique libre (cfDNA) par calcul du ratio cfDNA/ICE (Grewis) afin de s'assurer de ne mesurer que les fluctuations physiologique et/ou pathologique de cet ADN circulant chez un sujet d'intérêt en comparaison d'autres points d'analyse au cours du temps ou comparativement à un témoin (sujet sain).

Par `fragment d'ADN exogène', on entend un fragment d'ADN d'une autre espèce que celle l'ADN circulant d'intérêt; on choisira préférentiellement un fragment d'ADN exogène non humain pour l'extraction d'ADN circulant génomique humain.

Par `ADN circulant', on entend l'ADN libre de toutes cellules. On parlera indifféremment d'ADN libre de toutes cellules ou d'ADN circulant (ADNcf) dans le reste de la description. Selon un mode particulier, il s'agira d'ADN circulant humain, en particulier d'ADN circulant d'un sujet d'intérêt.

Par `sujet d'intérêt', on entend un sujet distinct d'un sujet normal ou sain. Le sujet d'intérêt selon l'invention est un sujet susceptible de libérer des ADNs libres de toutes cellules, en particulier lié à des phénomènes d'apoptose, de nécrose et/ou dans une moindre mesure à de la sécrétion active. Un tel sujet d'intérêt selon l'invention revendiquée est un sujet atteint ou qui développe un cancer ou une condition clinique pathologique choisie notamment parmi un AVC ou un infarctus myocardique, une insuffisance rénale aiguë, une cytolyse hépatique, un traumatisme, un rejet de greffe, ou un sujet soumis à un traitement médical tel que biopsie, chirurgie, radiothérapie, chimiothérapie, immunothérapie, ou principe actif (chimique ou biologique).

Le procédé de détection et quantification d'ADN circulant mettant en oeuvre une étape d'extraction dans laquelle sont ajoutés des calibrants ou standards selon les modes de réalisation utiles à la compréhension de l'invention décrits ci-après, présente les avantages suivants en fonction des modes de réalisations décrits ci-après :
- Un recouvrement supérieur des ADNs fragmentés pour les applications sensibles ;
- Le contrôle du rendement d'extraction par standardisation (contrôle interne exogène ICE grâce au premier fragment d'ADN exogène de 50-2000 paires de bases) ;
- Eventuellement le contrôle de l'efficacité de l'extraction pour les tailles proches de 100 paires de bases (LED grâce au second fragment optionnel d'ADN exogène de 50-150 paires de bases);
- Une optimisation des rendements à l'élution des ADN circulants (en présence d'albumine ou gélatine); et/ou
- Une concentration de l'élution des ADN circulants (ultrafiltration),
- Une co-amplification selon l'invention du contrôle interne exogène et d'une séquence ADN génomique (ex : ADN muté notamment en oncologie ou ADN du donneur dans le cas d'une transplantation).

Elle est aussi avantageuse, notamment pour les modes de réalisation préférés de l'invention, de par sa praticité et le gain de temps grâce des accessoires surmontant les colonnes d'extraction (permettant de charger la totalité du mélange échantillons/solution d'extraction en une fois, ce qui limite les contaminations croisées), son contrôle de qualité interne (milieu de culture lyophilisé comme échantillon témoin), sa reproductibilité (albumine, gélatine ou substituts à l'étape d'élution), et la concentration des ADN circulants (ultrafiltration).

### RESUME DE L'INVENTION

Les objets de l'invention sont définis dans les revendications 1 à 13.

Certains des modes de réalisation qui suivent ne sont pas couverts par le texte des revendications mais sont considérés comme utiles à la compréhension de l'invention.

Un premier mode de réalisation utile à la compréhension de l'invention est un procédé de détection et/ou de quantification d'ADN libre de toutes cellules à partir d'un échantillon de fluide biologique d'un sujet d'intérêt, comprenant au moins :
(i) une étape d'extraction d'ADN libre de toutes cellules à partir d'un échantillon de fluide biologique dans lequel on a ajouté au moins
   a) une quantité efficace d'un premier fragment d'ADN exogène de 50-2000 paires de bases, de préférence 50-200 paires de bases selon l'invention revendiquée, de préférence 60-160 paires de bases, de préférence encore 70-150 paires de bases et mieux de 80-140 paires de bases (ICE), et
   b) éventuellement une quantité efficace d'un second fragment d'ADN exogène de 50-150 paires de bases (LED) de séquence distinct du premier fragment d'ADN exogène;
(ii) une étape d'amplification et quantification de l'ADN libre de toutes cellules et des fragments d'ADN exogènes ICE et/ou LED extraits à l'étape (i), et
(iii) une étape de standardisation de la quantité d'ADN libre de toutes cellules extrait et éventuellement de son profil en taille comprenant :
   - le calcul d'un premier ratio du nombre de copies d'ADN libre de toutes cellules sur le nombre de copies de fragment d'ADN exogène de 50-2000 paires de bases, de préférence 50-200 paires de bases selon l'invention revendiquée, de préférence 60-160 paires de bases, de préférence encore 70-150 paires de bases et mieux de 80-140 paires de bases (ICE), et
   - éventuellement le calcul d'un second ratio du nombre de copies du premier fragment d'ADN exogène de 50-2000 paires de bases (ICE) sur le nombre de copies du second fragment d'ADN exogène de 50-150 paires de base (LED).

Par `quantité efficace' de fragment d'ADN exogène, on entend une quantité (ou un nombre de copies pour une taille donnée du fragment) d'ADN exogène par ml d'échantillon de fluide biologique permettant la mise en oeuvre de l'étape de standardisation de la quantité d'ADN circulant extrait et éventuellement de son profil de taille.

Selon un mode particulier, la quantité d'ADN exogène par ml d'échantillon de fluide biologique est d'au moins 1fg, voire 2 fg par ml d'échantillon (soit 10000 à 20000 copies par ml d'échantillon de plasma pour un fragment de 110 paires de bases), en particulier va de 1fg ou 2 fg à 2ng par ml d'échantillon.

Ces concentrations en calibrants, dans des zones proches voire inférieures aux concentrations en copies de gènes d'ADN libre de toutes cellules d'un sujet normal (ex : inférieures à 8ng/ml), permet de travailler aux valeurs physiologiques d'un sujet sain afin de mesurer précisément les fluctuations.

L'étape (i) d'extraction d'ADN libre de toutes cellules à partir d'un échantillon de fluide biologique comprendra généralement successivement une étape de protéolyse dudit échantillon biologique, une étape d'isolation de l'ADN libre circulant, et une étape d'élution, éventuellement précipitation et éventuellement concentration de l'ADN libre circulant.

L'étape (ii) d'amplification et quantification de l'ADN libre de toutes cellules extrait à l'étape précédente sera de préférence selon l'invention réalisée par une méthode de digital PCR, Q-PCR et/ou NGS.

Selon un mode particulier de l'invention, on co-amplifie dans un même puits le fragment d'ADN exogène et une séquence humaine détectée par ddPCR ou QPCR Taqman ou NGS. On parlera de `co-amplification' ou co-détection.

Le procédé selon l'invention permet également de faire du multiplexage en dPCR ou NGS de ce calibrant (ICE) et d'anomalies géniques (mutations, amplifications...) afin de suivre l'évolution de la concentration des anomalies au cours du temps ou comparativement à un sujet de référence (sujet sain, témoin).

Par `sujet de référence (témoin, sujet sain)', on entend un sujet 'normal', distinct du sujet d'intérêt, c'est-à-dire non susceptible d'être atteint ou de développer une indication clinique telle que décrite pour le sujet d'intérêt.

L'étape (iii) de standardisation, caractéristique du procédé de détection et quantification de l'invention, permet de mesurer de façon simple et fiable la quantité d'ADN libre circulant extrait et ses variations au cours du temps (nouvelles extractions et quantifications à partir d'un échantillon de fluide biologique du même sujet à des temps définis t0, t1, t2...) ou comparativement à un sujet de référence (témoin, sujet sain), et éventuellement son profil en taille pour vérifier la reproductibilité de l'extraction d'ADN circulant à la zone de taille spécifique, en particulier de l'ADN circulant de taille autour de 100 paires de bases.

Un autre mode de réalisation utile à la compréhension de l'invention porte sur l'utilisation du procédé de détection et/ou de quantification d'ADN libre de toutes cellules à partir d'un échantillon de fluide biologique d'un sujet d'intérêt selon l'invention, dans une méthode d'analyse d'un échantillon biologique d'un sujet d'intérêt susceptible de libérer des ADNs libres de toutes cellules, en particulier un sujet d'intérêt atteint ou susceptible de développer un cancer ou une condition clinique choisie notamment parmi un AVC ou un infarctus myocardique, une insuffisance rénale aiguë, une cytolyse hépatique, un traumatisme, une chirurgie, un rejet de greffe, ou un sujet soumis à un exercice musculaire intensif, ou un sujet soumis à un traitement médical tel que biopsie, chirurgie, radiothérapie, chimiothérapie, immunothérapie, ou principe actif.

Un autre mode de réalisation utile à la compréhension de l'invention porte encore sur un procédé *in vitro* d'analyse, en particulier de diagnostic, pronostic, théranostic, ou de suivi de l'évolution d'un état physiologique spécifique d'un sujet d'intérêt susceptible de libérer de l'ADN circulant, en particulier un sujet d'intérêt atteint ou susceptible de développer un cancer ou une condition clinique choisie notamment parmi un AVC ou un infarctus myocardique, une insuffisance rénale aiguë, une cytolyse hépatique, un traumatisme, une chirurgie, un rejet de greffe, ou un sujet soumis à un exercice musculaire intensif, ou un sujet soumis à un traitement médical tel que biopsie, chirurgie, radiothérapie, chimiothérapie, immunothérapie, ou principe actif., comprenant les étapes suivantes :
(i) à un temps t0, détection et/ou de quantification d'ADN libre de toutes cellules à partir d'un échantillon de fluide biologique d'un sujet d'intérêt selon le procédé de l'invention tel que sus-décrit;
(ii) à un temps t1, reproduction de l'étape (i), et
   a. comparaison du ratio (Grewis) du nombre de copies d'ADN circulant sur nombre de copies de fragment d'ADN exogène de 50-2000 paires de bases, de préférence 50-200 paires de bases selon l'invention, de préférence 60-160 paires de bases, de préférence encore 70-150 paires de bases et mieux de 80-140 paires de bases (ICE) obtenu au temps t1 par rapport au même ratio obtenu au temps t0.

Les étapes (ii) et (iii) pouvant être reproduites autant de fois que nécessaire (par exemple à t2, t3, t4...) pour suivre l'évolution d'un état physiologique spécifique d'un sujet d'intérêt, et par voie de conséquence l'effet d'un traitement médical (chirurgie, médicaments, radiothérapies,...) sur ledit sujet.

Ces mesures pourront être faites facilement à partir d'un échantillon de sang collecté sur ledit sujet (prise de sang).

La durée de temps écoulé entre deux temps de mesure (t0 et t1, t1 et t2...) pourra généralement représenter une ou quelques heures, jours, semaines, mois ou années, en fonction de l'état physiologique du sujet d'intérêt et de l'objectif recherché par la quantification de l'ADN circulant à partir d'un échantillon de fluide biologique dudit sujet.

L'homme du métier saura ainsi adapter cette durée de temps écoulé entre deux temps de mesure en fonction de l'état physiologique du sujet d'intérêt et des éventuels traitements médicaux suivis afin de définir les t1, t2 et temps suivants par rapport à un t0 donné.

Le procédé in vitro d'analyse selon l'invention est destiné notamment à :
- analyser les fluctuations d'ADN libre de toutes cellules pour suivre la réponse et/ou résistance à un traitement médical tel que biopsie, chirurgie, radiothérapie, chimiothérapie, immunothérapie, ou principe actif, ou
- analyser les fluctuations d'ADN libre de toutes cellules pour suivre l'apparition d'un rejet de greffe, ou
- analyser les fluctuations d'ADN libre de toutes cellules pour décider de l'intérêt de procéder à une analyse subséquente d'anomalies génomiques, ou
- suivre l'évolution de la concentration en ADN muté, ou
- suivre l'évolution de la concentration en ADN du donneur dans le cas particulier d'un sujet d'intérêt transplanté susceptible d'avoir un rejet de greffe.

Alternativement, un mode de réalisation utile à la compréhension de l'invention porte sur un procédé *in vitro* de diagnostic, pronostic, théranostic d'un sujet d'intérêt susceptible de libérer de l'ADN circulant, en particulier un sujet d'intérêt atteint ou susceptible de développer un cancer ou une condition clinique choisie notamment parmi un AVC ou un infarctus myocardique, une insuffisance rénale aiguë, une cytolyse hépatique, un traumatisme, une chirurgie, un rejet de greffe, ou un sujet soumis à un exercice musculaire intensif, ou un sujet soumis à un traitement médical tel que biopsie, chirurgie, radiothérapie, chimiothérapie, immunothérapie, ou principe actif, comprenant les étapes suivantes :
(i) à un temps t, détection et/ou de quantification d'ADN libre de toutes cellules à partir d'un échantillon de fluide biologique d'un sujet d'intérêt selon l'une quelconque des revendications 1 à 5;
(ii) comparaison du ratio (Grewis) du nombre de copies d'ADN circulant sur nombre de copies de fragment d'ADN exogène de 50-2000 paires de bases, de préférence 50-200 paires de bases selon l'invention, de préférence 60-160 paires de bases, de préférence encore 70-150 paires de bases et mieux de 80-140 paires de bases (ICE) obtenu au temps t par rapport au même ratio obtenu chez un sujet de référence (sujet sain, témoin).

Un autre mode de réalisation utile à la compréhension de l'invention est un kit ou ensemble d'extraction d'ADN libre de toutes cellules à partir d'un échantillon de fluide biologique d'un sujet d'intérêt, comprenant :
(i) Au moins un premier fragment d'ADN exogène de 50-2000 paires de bases, de préférence 50-200 paires de bases selon l'invention, de préférence 60-160 paires de bases, de préférence encore 70-150 paires de bases et mieux de 80-140 paires de base (ICE);
(ii) Des tampons et enzymes, en particulier un tampon de protéolyse, une enzyme de protéolyse, un tampon de fixation, un tampon de lavage, et un tampon d'élution de l'ADN libre de toutes cellules,
(iii) Des dispositifs permettant l'isolation de l'ADN libre de toutes cellules, en particulier des colonnes d'extractions à membrane de silice et avantageusement des colonnes d'extractions surmontées d'accessoires de charge, de préférence des entonnoirs,
(iv) Des plaques multi-puits, en particulier des plaques 24-puits DeepWell de 25 mL
(v) Des tubes à vis, de préférence de 1 ,5ml, et
(vi) Une notice d'utilisation,
ledit fragment d'ADN exogène ICE étant fourni dans ledit kit ou apporté séparément audit kit, formant un ensemble d'extraction.

Selon un mode particulier, les accessoires de charge utilisés à l'étape (iii) sont des accessoires surmontant les colonnes d'extraction et présentant au moins un diamètre supérieur au diamètre de ladite colonne, permettant de charger en une fois la totalité du mélange échantillon/solution d'extraction. En particulier, de tels accessoires permettent de charger dans la colonne des volumes allant de 0,5 à 100ml, en particulier de 1ml à 36ml, préférentiellement de 1ml à 14ml. Selon un mode particulier, le volume sera de 0,5 à 8ml, notamment de 0,5 à 4ml.

Ces accessoires peuvent avoir différentes formes. Selon un mode particulier et préféré, on utilisera comme accessoires permettant de charger la totalité du mélange échantillon/solution d'extraction, des entonnoirs.

Par `solution d'extraction' on entend la solution comprenant les tampons et les enzymes nécessaires à l'étape d'extraction de l'ADN circulant.

Selon un mode particulier, le kit d'extraction d'ADN libre de toutes cellules selon l'invention est utilisé en association avec un kit d'amplification et de quantification d'ADN libre de toutes cellules, fournis ensemble ou séparément.

Le kit d'amplification et de quantification d'ADN libre de toutes cellules comprendra généralement au moins :
(i) des amorces sens et antisens spécifiques respectivement de l'ADN libre de toutes cellules du sujet d'intérêt et du fragment d'ADN exogène tels que définis dans l'invention, pour l'étape d'amplification,
(ii) des sondes marquées respectivement pour l'ADN libre de toutes cellules du sujet d'intérêt et pour le fragment d'ADN exogène tels que définis dans l'invention, pour l'étape de détection et quantification,
(iii) des tampons de réaction,
(iv) des plaques multi-puits et
(v) une notice d'utilisation.

Un autre mode de réalisation utile à la compréhension de l'invention concerne l'utilisation d'au moins un premier fragment d'ADN exogène de 50-2000 paires de bases, de préférence 50-200 paires de bases selon l'invention, de préférence 60-160 paires de bases, de préférence encore 70-150 paires de bases et mieux de 80-140 paires de bases (ICE), pour son utilisation dans la standardisation d'une méthode d'extraction et de quantification d'ADN libre de toutes cellules et/ou dans une méthode d'analyse des fluctuations d'ADN libre de toutes cellules chez un sujet d'intérêt dans le temps par mesure du ratio (Grewis) du nombre de copies d'ADN libre de toutes cellules sur le nombre de copies du premier fragment d'ADN exogène de 50-2000 paires de bases, de préférence 50-200 paires de bases selon l'invention, de préférence 60-160 paires de bases, de préférence encore 70-150 paires de bases et mieux de 80-140 paires de bases (ICE).

Ledit fragment d'ADN exogène peut avantageusement être utilisé avec tout kit d'extraction et/ou de quantification d'ADN libre de toutes cellules.

### DESCRIPTION DETAILLEE DE L'INVENTION

Les objets de l'invention sont définis dans les revendications 1 à 13.

Certains des modes de réalisation qui suivent ne sont pas couverts par le texte des revendications mais sont considérés comme utiles à la compréhension de l'invention.

### PROCEDE DE DETECTION ET/OU QUANTIFICATION D'ADN CIRCULANT

Un premier mode de réalisation utile à la compréhension de l'invention concerne un procédé de détection et/ou de quantification d'ADN libre de toutes cellules à partir d'un échantillon de fluide biologique d'un sujet d'intérêt, comprend au moins :
(i) une étape d'extraction d'ADN libre de toutes cellules à partir d'un échantillon de fluide biologique dans lequel on a ajouté au moins une quantité efficace d'un premier fragment d'ADN exogène de 50-2000 paires de bases, de préférence 50-200 paires de bases selon l'invention, de préférence 60-160 paires de bases, de préférence encore 70-150 paires de bases et mieux de 80-140 paires de bases (ICE);
(ii) une étape d'amplification et quantification de l'ADN libre de toutes cellules et du fragment d'ADN exogène ICE extrait à l'étape (i), et
(iii) une étape de standardisation de la quantité d'ADN libre de toutes cellules extrait comprenant le calcul d'un premier ratio (Grewis) du nombre de copies d'ADN libre de toutes cellules sur le nombre de copies du premier fragment d'ADN exogène de 50-2000 paires de bases, de préférence 50-200 paires de bases, de préférence 60-160 paires de bases, de préférence encore 70-150 paires de bases et mieux de 80-140 paires de bases (ICE).

Selon l'invention, le sujet d'intérêt est un sujet atteint ou qui développe un cancer ou une condition clinique choisie parmi un AVC ou un infarctus myocardique, une insuffisance rénale aiguë, une cytolyse hépatique, un traumatisme, un rejet de greffe, ou un sujet soumis à un traitement médical tel que biopsie, chirurgie, radiothérapie, chimiothérapie, immunothérapie, ou principe actif (chimique ou biologique).

Selon un mode particulier de l'invention, le sujet d'intérêt est atteint ou développe un cancer. Selon un autre mode particulier de l'invention, le sujet d'intérêt est susceptible d'avoir un rejet de greffe.

Selon un autre mode particulier de l'invention, le sujet d'intérêt est soumis à un traitement médical choisi parmi une biopsie, une chirurgie, une radiothérapie, une chimiothérapie, une immunothérapie, ou un principe actif (chimique ou biologique).

Selon un mode particulier de l'invention, le fluide biologique est choisi dans le groupe constitué par le sang total, le sérum, le plasma, l'urine, la salive, l'effluent de la moelle osseuse, la lymphe, le liquide céphalorachidien, le liquide lacrymal, la sueur, le lait, l'humeur aqueuse, le liquide synovial, le liquide pleural, liquide péritonéal, liquide amniotique, la bile, liquide séminales, expectorations.

De préférence selon l'invention, le fluide biologique est le plasma.

Les échantillons de plasmas sont généralement préparés à partir de sang complet.

Afin de limiter la libération d'ADN cellulaire (cellules circulantes) provoquant la dilution de l'ADN libre de toutes cellules, on utilisera de préférence des protocoles incluant deux centrifugations. Selon un mode préféré, le sang total est collecté, par exemple sur tube des tubes Cell-Free DNA BCT^{®} (Streck), les tubes Roche (Ariosa) et EDTA K3 selon les recommandations des fournisseurs.

On réalisera avantageusement :
- une 1^{er} centrifugation (1200-1600g) à température ambiante (20°C +/- 5°C) réalisée dans une période de 6h maximum après le prélèvement ; le plasma (sumageant) est récupéré sans prélever la galette de cellules en séparation entre le plasma et les hématies ;
- une 2^{ème} centrifugation (3000-16000g) à température ambiante (20°C +/- 5°C) ; le plasma est aspiré sans prélever le culot formé.

Quels que soient les tubes précités, le plasma peut être conservé à -20°C pour une durée inférieure ou égale à 1 mois ou -80°C pour des durées supérieures à 1 mois.

Les principales étapes du procédé selon les modes de réalisation utile à la compréhension de l'invention décrits sont détaillées ci-après.

### Extraction d'ADN circulant

En particulier, l'étape d'extraction (i) selon l'invention comprendra les étapes suivantes :
(i1) Protéolyse dudit échantillon de fluide biologique d'un sujet d'intérêt après ajout dans ledit échantillon d'au moins une quantité efficace d'un premier fragment d'ADN exogène de 50-200 paires de bases, de préférence 60-160 paires de bases, de préférence encore 70-150 paires de bases et mieux de 80-140 paires de bases (ICE) et éventuellement une quantité efficace d'un second fragment d'ADN exogène de 50-150 paires de bases (LED) ,
(i2) Isolation de l'ADN libre de toutes cellules, notamment par affinité sur un support de membrane, de préférence un support de membrane de silice dans une colonne d'extraction,
(i3) Elution de l'ADN libre de toutes cellules isolé à l'étape (i2) dans une phase aqueuse,
(i4) Eventuellement précipitation de l'ADN libre de toutes cellules,
(i5) Avantageusement concentration de l'ADN libre de toutes cellules,
(i6) Eventuellement conservation et/ou stockage dudit ADN libre de toutes cellules.

Selon un mode particulier, l'étape d'extraction (i) ne comprend pas d'étape (i4) de précipitation.

Selon un mode particulier de l'invention, le procédé de l'invention comprend une co-amplification d'un ADN libre de toutes cellules, d'un fragment exogène ICE et d'une séquence d'ADN génomique choisie parmi une séquence d'ADN muté ou une séquence d'ADN `donneur'.

Par `séquence d'ADN muté', on entend selon l'invention une séquence présentant une mutation. Par exemple dans le cas d'un cancer on cherchera à détecter/quantifier des mutations activatrices ou de résistance à une thérapie ciblée. Dans le cas d'un cancer de la prostate, on cherchera à détecter/quantifier des mutations du gène codant pour le récepteur aux androgènes de résistance à un traitement par Abiraterone ou autres thérapies ciblées. Dans le cas d'un cancer du poumon non à petites cellules (CBNPC), on cherchera à détecter/quantifier des mutations du gène codant pour l'EGFR activatrices ou de résistance à l'Osimertinib ou autres thérapie ciblée.

Par `séquence d'ADN `donneur" ou `séquence d'ADN du donneur', on entend selon l'invention une séquence d'ADN du donneur (cas de transplantation) par opposition à l'ADN du receveur, en ciblant SNP ou autres séquences HLA.

Chacune des étapes de la méthode d'extraction est précisée dans la description qui suit.

### (i1) Protéolyse dudit échantillon biologique

Le ou les standards (fragments d'ADN exogènes précités), caractérisant le procédé d'extraction, d'amplification et de quantification d'ADN circulant de l'invention, sont ajoutés à l'échantillon de fluide biologique d'un sujet d'intérêt avant l'étape de protéolyse.

Selon un mode de réalisation utile à la compréhension de l'invention, on ajoute au moins le premier fragment d'ADN exogène de 50-2000 paires de bases, de préférence 50-200 paires de bases selon l'invention, de préférence 60-160 paires de bases, de préférence encore 70-150 paires de bases et mieux de 80-140 paires de bases (ICE) et éventuellement le second fragment d'ADN exogène de 50-150 paires de bases (LED) . Selon un mode particulier, on utilisera leur mélange. Selon un autre mode particulier et préféré, on utilisera seulement le premier fragment ICE à condition qu'il soit de petite taille, c'est-à-dire de 50 à 200 paires de bases selon l'invention, de préférence 60 à 160 paires de base, de préférence 70 à 150 paires de bases, et mieux de 80 à 140 paires de bases. A ces petites tailles, le fragment ICE joue ainsi avantageusement également le rôle du fragment LED.

Le premier fragment d'ADN exogène de 50-2000 paires de bases, de préférence de 50-200 paires de bases selon l'invention, de préférence 60-160 paires de bases, de préférence encore 70-150 paires de bases et mieux de 80-140 paires de bases sert au contrôle interne exogène (ICE).

L'homme du métier définira la taille du premier fragment d'ADN exogène à utiliser en fonction du sujet d'intérêt dont on souhaite quantifier l'ADN circulant et en fonction de la technique analytique (quantification) disponible.

Selon l'invention, ce premier fragment d'ADN exogène a une taille allant de 50 à 200 paires de bases, de préférence 60 à 160 paires de base, de préférence 70 à 150 paires de bases, et mieux de 80 à 140 paires de bases.

Selon un autre mode de réalisation non couvert par le texte des revendications, le premier fragment d'ADN exogène (ICE) est un fragment de préférence de 200 à 1000 paires de base, de préférence encore de 250 à 350 paires de bases.

Selon un mode particulier de réalisation non couvert par le texte des revendications, le premier fragment d'ADN exogène est un fragment d'ADN non humain de 300 paires de bases.

Selon un mode particulier et préféré de l'invention, le premier fragment d'ADN exogène est un fragment d'ADN non humain de 110 paires de bases.

Ce standard inter-essais (ICE) permet de standardiser la quantité d'ADN circulant extrait et ses variations inter-essais (et notamment dans le temps pour un même sujet d'intérêt) ou comparativement par rapport à la quantité d'ADN circulant extrait chez un sujet de référence (sujet sain, témoin) en calculant le ratio (Grewis) du nombre de copies d'ADN circulant sur le nombre de copies de premier fragment d'ADN exogène de 50-2000 paires de bases, de préférence de 50-200 paires de bases selon l'invention, de préférence 60-160 paires de bases, de préférence encore 70-150 paires de bases et mieux de 80-140 paires de bases (ICE). Ces valeurs sont obtenues après extraction, amplification et quantification desdits fragments d'ADN exogène, en parallèle de l'amplification et de la quantification de l'ADN circulant, comme décrit ci-après dans la description.

La valeur du ratio est exprimée en Unité arbitraire (Grewis). Cette approche permet ainsi de s'affranchir de tout calcul ramenant à des volumes d'éluat.

Selon un mode de réalisation utile à la compréhension de l'invention, le premier fragment d'ADN exogène de 50-2000 paires de base peut être utilisé seul ou en combinaison avec le second fragment d'ADN exogène de 50-150 paires de base, distinct du premier fragment.

Le second fragment d'ADN exogène de 50-150 paires de base, distinct du premier fragment, sert au contrôle des fragments de petite taille (LED).

Selon un mode particulier, le second fragment d'ADN exogène est un fragment d'ADN exogène de 80-120 paires de bases.

Selon un mode particulier, le second fragment d'ADN exogène est un fragment d'ADN non humain de 110 paires de bases.

Ce second fragment d'ADN exogène (LED) permet de vérifier la reproductibilité d'extraction des ADN circulants à la zone de taille spécifique, autrement dit standardiser le profil en taille de l'ADN circulant extrait, et parer à l'influence de la composition chimique/biologique du plasma sur l'efficacité de l'extraction des fragments de petites tailles. On calcule le ratio du nombre de copies du premier fragment d'ADN exogène de 50-2000 paires de bases (ICE) sur le nombre de copies du second fragment d'ADN exogène de 50-150 paires de bases (LED).

Selon un mode particulier et préféré de l'invention, on utilisera seulement un premier fragment ICE de 50 à 200 paires de bases, de préférence 60 à 160 paires de base, de préférence 70 à 150 paires de bases, et mieux de 80 à 140 paires de bases, qui jouera également le rôle du fragment LED.

L'étape de protéolyse ou hydrolyse des protéines sous l'action d'enzymes est généralement réalisée en présence d'un tampon et d'enzymes.

Selon un mode particulier, on utilisera une enzyme (protéinase K) et un tampon de lyse (LYS). L'homme du métier déterminera les quantités d'enzyme et de tampon de lyse à utiliser en fonction du volume d'échantillon à traiter. Selon un mode particulier, le mélange, une fois agité sous vortex, est mis à incuber à une température d'activation de l'enzyme, en particulier une température de 56°C +/- 5°C pendant 30 minutes à 1 heure sous agitation.

Selon un mode particulier, le tampon de lyse contient des sels chaotropes (Hydrochloride de guanidinium) ; ces sels apportent le chao dans les macromolécules et permettent d'écarter les molécules d'eau de la silice et des ADNs une fois les molécules chargées. Il peut se créer un pont favorable à la fixation des ADNs et des standards sur la silice
Selon un mode préféré, le procédé de détection et/ou de quantification d'ADN libre de toutes cellules selon un mode de réalisation utile à la compréhension de l'invention, met en outre en oeuvre dans l'étape d'extraction (i) au moins un échantillon témoin constitué de préférence par un lyophilisat de milieu de culture de lignée cellulaire mutée ou non, à réhydrater pour mimer l'échantillon de fluide biologique.

Par lignée cellulaire mutée, on entend notamment une lignée cellulaire dont le génome présente des modifications telles que des SNV pour Single Nucléotide Variant, délétions, insertions, CNV pour Copy Number Variation,...)

Ce contrôle de qualité du rendement d'extraction permet de vérifier la reproductibilité inter-essais de l'extraction en calculant les 2 mêmes ratios sur cet échantillon témoin, soit respectivement le ratio du nombre de copies d'ADN circulant sur nombre de copies du premier fragment d'ADN exogène de 50-2000 paires de bases et le ratio du nombre de copies du premier fragment d'ADN exogène de 50-2000 paires de bases (ICE) sur le nombre de copies du seconde fragment d'ADN exogène de 50-150 paires de bases (LED), qui ne doivent pas varier d'une série d'extraction à l'autre.

L'échantillon témoin selon l'invention subit les mêmes réactions que l'échantillon de fluide biologique du sujet d'intérêt dont on souhaite extraire l'ADN circulant, en particulier l'ajout des standards (fragments d'ADN exogène ICE et LED, extraction, amplification, quantification et contrôle).

### (i2) Isolation de l'ADN circulant

L'isolation de l'ADN circulant peut notamment être réalisée selon l'une des méthodes suivantes :
a. Par affinité de l'ADN circulant pour un support (membrane de silice, billes magnétiques,...), de préférence un support de membrane de silice, ou
b. Par méthode d'extraction entre 2 phases liquides non miscibles (Phenol/chloroformes, Trizol, méthodes Piotr Chomczynski et Nicoletta Sacchi)
c. Par gradient de densité (ex : chlorure de césium...).

Selon un mode particulier de l'invention, on utilisera la méthode d'affinité sur un support de membrane, de préférence un support de membrane de silice dans une colonne d'extraction. De préférence, on utilise des colonnes MIDI selon les recommandations du fournisseur, en présence de tampons d'activation et de fixation.

Selon un mode particulier, l'étape d'isolation et de fixation de l'ADN circulant sur le support de membrane de silice consiste à :
- appliquer le tampon d'activation des membranes de silice dans chaque colonne MIDI, incuber puis appliquer le vide (-0,4 bar);
- ajouter un tampon de fixation par ml de plasma dans chaque échantillon lysé ;
- mélanger le contenu du tube par un minimum de 5 aspirations/refoulements ;
- mettre en place les entonnoirs sur chaque colonne ;
- appliquer le mélange échantillon lysé dans les colonnes MIDI ;
- appliquer le vide à -0.4 bar pendant 5 minutes en s'assurant que tout le lysat est bien passé à travers la colonne.

Les tampons d'activation et de fixation sont les tampons utilisés dans les protocoles d'extraction usuels.

Selon un mode particulier, on utilisera un tampon de fixation Hydrochloride de guanidinium-éthanol.

Selon un mode préféré du procédé de détection et/ou de quantification d'ADN libre de toutes cellules selon l'invention, la colonne d'extraction à l'étape (i2) d'isolation de l'ADN libre de toutes cellules est surmontée d'un accessoire de charge, de préférence un entonnoir permettant de charger la totalité du mélange échantillon/solution d'extraction soit un volume allant de préférence de 1ml à 14ml.

Cette configuration permet de manipuler en un seul pipetage la totalité (manuel) ou la moitié (automatisé) du mélange plasma- tampon de protéolyse- solution de fixation.

L'étape d'isolation et de fixation d'ADN circulant est suivie d'une étape de lavage de la membrane selon les recommandations classiques du fournisseur (plusieurs cycles de lavage, application du vide à -0,4 bar pendant 5 minutes).

### (i3) Elution de l'ADN circulant

Selon un mode particulier, avant de procéder à l'élution de l'ADN circulant, on applique le vide, par exemple au minimum -0.6 bar pendant 10 minutes, pour sécher complètement la membrane de toute trace d'éthanol.

On ajoute ensuite un tampon d'élution selon les recommandations du fournisseur.

Selon un mode préféré du procédé de détection et/ou de quantification d'ADN libre de toutes cellules selon l'invention, l'étape (i3) d'élution d'ADN libre de toutes cellules isolé à l'étape (i2) est réalisée en présence d'un tampon d'élution supplémenté en albumine, gélatine, ou substituts.

Ceci permet avantageusement d'augmenter le rendement d'extraction et de stabiliser la reproductibilité du volume élué.

Selon un premier mode, l'étape d'élution est réalisée en présence d'un tampon d'élution supplémenté en albumine, telle que la BSA (Bovine Albumine Sérum), de préférence à 0,5 mg/ml d'éluat.

Selon un autre mode préféré, l'étape d'élution est réalisée en présence d'un tampon d'élution supplémenté en gélatine, telle que celle commercialisée par SIGMA sous la dénomination commerciale Prionex^{®}, de préférence utilisé à 1%.

### (i4) Eventuellement précipitation et (i5) concentration de l'ADN circulant

Selon un mode particulier, le procédé d'extraction ne comprend pas d'étape (i4) de précipitation.

Selon un mode préféré du procédé de détection et/ou de quantification d'ADN libre de toutes cellules selon l'invention, l'étape (i5) de concentration de l'ADN pur libre de toutes cellules est réalisée sur une plaque de concentration par ultrafiltration.

Cette étape de concentration pourra être réalisée par exclusion de la phase aqueuse, et avantageusement sans ajout de sels chaotropes pour la fixation et le lavage alcoolique de l'ADN circulant.

Le volume à concentrer peut aller de 20-300µl, le volume à récupérer peut aller de 25-300µl.

### (i6) Conservation et/ou stockage de l'ADN circulant

Les ADN circulants obtenus doivent être conservés à 5°C ± 3°C si la dPCR (Digital PCR) ou ddPCR (Droplet Digital PCR) ou Q-PCR (Quantitative PCR) est réalisée en suivant, sinon les ADN circulants doivent être stockés à -80 °C pour une conservation plus longue.

Selon un mode particulier préféré de l'invention, l'étape d'extraction d'ADN libre de toutes cellules est réalisée selon les étapes suivantes :
(i1) Protéolyse du plasma, et en parallèle de l'échantillon témoin (CQI), dans lesquels on a ajouté le ou les standards (fragments d'ADN exogène ICE et LED), avec de la Protéinase K et des tampons de lyse contenant des sels chaotropes Hydrochloride de guanidinium ;
(i2) isolation d'ADN libre de toutes cellules sur une membrane de silice, en présence d'un tampon de fixation Hydrochloride de guanidinium-éthanol, ajouté en grand volume afin de fixer les DNA à la silice, le % d'éthanol influant sur la taille des fragments retenus. Le volume est ajouté sur la colonne de silice équipée d'un entonnoir amovible en 1 étape en manuel et 2 étapes en automatique. 3 étapes de lavage sont nécessaires à l'élimination des inhibiteurs et des sels. Ensuite un séchage de 10minutes permet d'éliminer l'éthanol présent sur la membrane de silice ;
(i3) élution de l'ADN libre par une première étape dans laquelle l'ADN libre est désorbé grâce à un tampon sans EDTA faiblement tamponné a pH 8,5. Avantageusement, l'étape d'extraction selon l'invention ne prévoit pas d'addition d'ARN entrainant qui pourrait interférer avec certaines applications ultérieures comme l'amplification avec la méthode NGS. L'élution est de préférence réalisée en tampon supplémenté en albumine ou gélatine qui optimise le volume et le rendement d'ADN extrait.
(i4) et (i5) l'éluat peut être concentré par ultrafiltration si nécessaire, pour des applications en aval.

Des procédés avec étape d'extraction pour des tubes collecte de sang Cell-Free DNA BCT^{®} de Streck ont également été développés. Les réactifs stabilisants des tubes BCT nécessitent une protéolyse prolongée afin d'isoler efficacement les ADN circulants.

### Amplification et quantification de l'ADN circulant

Cette étape d'amplification et de quantification de l'ADN libre de toutes cellules et du ou des fragments d'ADN exogènes (standards ICE et éventuellement LED) extraits à l'étape précédente, peut être réalisée par les méthodes bien connues de l'homme du métier.

Selon un mode particulier, le procédé de l'invention permet une co-amplification, en particulier dans un même puits, d'un fragment exogène ICE et d'une séquence génomique présentant une mutation ou d'une séquence ADN du donneur (transplantation), permettant de suivre l'évolution de la concentration en ADN muté ou d'ADN du donneur entre deux extractions dans le temps.

Selon des modes préférés de l'invention, elle pourra être réalisée par une méthode de digital PCR, de Q-PCR (PCR quantitative en temps réel) et/ou Nouvelles Générations de Séquenceurs à ADN (NGS), selon les préconisations des fournisseurs.

On utilisera préférentiellement la digital PCR (dPCR) pour sa capacité à multiplexer les amplifications, sa sensibilité, sa quantification absolue, sa haute résolution pour les variations de quantité obtenue grâce à la loi de Poisson.

On utilisera la Q-PCR en substitut dans le cas où la dPCR n'est pas disponible.

Enfin on utilisera plutôt le NGS en utilisant sa capacité de profondeur de lecture pour quantifier les ADN exogènes (ICE et LED) et l'ADN génomique.

Selon un mode particulier, les ADN seront quantifiés de manière absolue à l'aide d'un système dPCR comprenant :
- Un système d'amplification de l'ADN circulant extrait comprenant des amorces sens et antisens d'amplification d'une région du génome humain et des sondes spécifiques, avantageusement marquées par des fluorophores, de détection de l'ADN circulant amplifié ;
- Un système d'amplification du premier fragment d'ADN exogène (ICE) extrait comprenant des amorces sens et antisens d'amplification dudit premier fragment et des sondes spécifiques, avantageusement marquées par des fluorophores, de détection dudit premier fragment d'ADN exogène amplifié (ICE) ;
- Eventuellement un système d'amplification du second fragment d'ADN exogène (LED) extrait comprenant des amorces sens et antisens d'amplification dudit second fragment et des sondes spécifiques, avantageusement marquées par des fluorophores, de détection dudit second fragment d'ADN exogène amplifié (LED).

Les valeurs absolues quantifiées pour chaque ADN (ADN circulant, fragments d'ADN exogène ICE et éventuellement LED), respectivement extrait à partir d'un échantillon de fluide biologique d'un sujet d'intérêt, d'un sujet de référence (sujet sain, témoin) ou d'un échantillon témoin, permettront de calculer les ratios selon l'étape de standardisation de la quantité d'ADN libre de toutes cellules extrait et éventuellement de son profil en taille décrite ci-après.

### Standardisation de la quantité d'ADN libre de toutes cellules extrait

Selon un mode de réalisation utile à la compréhension de l'invention, l'étape de standardisation de la quantité d'ADN libre de toutes cellules extrait et éventuellement de son profil en taille, caractéristique du procédé de détection et de quantification d'ADN circulant comprend, pour les ADNs extraits de l'échantillon de fluide biologique et avantageusement en parallèle, pour les ADNs extraits de l'échantillon témoin :
- le calcul d'un premier ratio (Grewis) du nombre de copies d'ADN libre de toutes cellules sur le nombre de copies du premier fragment d'ADN exogène de 50-2000 paires de bases, de préférence de 50-200 paires de bases selon l'invention, de préférence 60-160 paires de bases, de préférence encore 70-150 paires de bases et mieux de 80-140 paires de bases (ICE), et
- éventuellement le calcul d'un second ratio du nombre de copies du premier fragment d'ADN exogène 50-2000 paires de bases (ICE) sur le nombre de copies du second fragment d'ADN exogène de 50-150 paires de base (LED),

On calcule ainsi après chaque extraction, amplification et quantification d'ADN, pour l'échantillon de fluide biologique (ex : plasma) et pour l'échantillon témoin, le Grewis (premier ratio) et l'on vérifie éventuellement l'efficacité de l'extraction des fragments de petites taille autour 80-120bases (second ratio).

La valeur du premier ratio (Grewis) sert de référence qui peut être comparée entre chaque extraction est ainsi définir les variations de quantité d'ADN circulant chez un sujet donné au cours du temps ou chez un sujet d'intérêt comparativement à une référence (sujet sain, témoin).

Si la valeur du premier ratio (Grewis) augmente entre deux extractions, cela signifie que la quantité d'ADN circulant chez le sujet a augmenté entre la première et la deuxième extraction.

Si la valeur du premier ratio (Grewis) diminue entre deux extractions, cela signifie que la quantité d'ADN circulant chez le sujet a diminué entre la première et la deuxième extraction. Si la valeur du premier ratio (Grewis) pour un sujet d'intérêt est supérieure au même ratio pour une référence (sujet sain, témoin), cela signifie que la quantité d'ADN circulant chez le sujet d'intérêt est plus importante, signe d'un phénomène d'apoptose, de nécrose et/ou de sécrétion active.

La valeur du second ratio ne doit en revanche pas varier d'une extraction à l'autre.

Les valeurs chiffrées n'ont pas d'importance ; on choisira par exemple une valeur du second ratio égale à 1, soit des proportions du premier fragment d'ADN exogène (ICE) et du second fragment d'ADN exogène (LED) identiques.

Si ce second ratio augmente, cela signifie que les ADN de petites tailles n'ont pas été extraits correctement, et que les résultats obtenus sur la quantification de l'ADN circulant sont biaisés.

### UTILISATIONS

Un autre mode de réalisation utile à la compréhension de l'invention porte sur l'utilisation d'un procédé de détection et/ou de quantification d'ADN libre de toutes cellules à partir d'un échantillon de fluide biologique d'un sujet d'intérêt selon l'invention, dans une méthode d'analyse d'un échantillon biologique d'un sujet d'intérêt susceptible de libérer des ADNs libres de toutes cellules, en particulier un sujet atteint ou susceptible de développer un cancer ou une condition clinique choisie notamment parmi un AVC ou un infarctus myocardique, une insuffisance rénale aiguë, une cytolyse hépatique, un traumatisme, une chirurgie, un rejet de greffe, ou un sujet soumis à un exercice musculaire intensif, ou un sujet soumis à un traitement médical tel que biopsie, chirurgie, radiothérapie, chimiothérapie, immunothérapie, ou principe actif (chimique ou biologique).

Ce procédé de détection et/ou de quantification d'ADN libre de toutes cellules pourra notamment être utilisé dans des méthodes d'analyse *in vitro* d'échantillons de fluide biologique (ex : plasma sanguin) de sujet d'intérêt pour des applications de diagnostic, pronostic, théranostic, ou de suivi de l'évolution d'un état physiologique spécifique d'un individu.

Selon un mode particulier préféré, ce procédé de détection et/ou de quantification d'ADN libre de toutes cellules sera utilisé pour le suivi de l'évolution d'un état physiologique spécifique d'un individu sous traitement, par exemple un traitement chirurgical, ou un traitement médicamenteux (ex : drogues), ou un traitement par radiothérapie ou tout autre procédé provoquant la mort cellulaire induisant la variation de la quantité d'ADN libre de toutes cellules.

A titre d'exemple, on pourra mesurer, pour un sujet d'intérêt susceptible de libérer de l'ADN circulant, par exemple un sujet atteint ou susceptible de développer un cancer, et à partir d'un échantillon de fluide biologique (plasma), à un temps t0 de sa vie, la valeur du premier ratio (Grewis).

Cette valeur à t0 peut avoir une valeur biologique de diagnostic d'une pathologie, pronostic d'un événement médical (métastase, reprise de la maladie, décès,...), en particulier comparativement à un sujet de référence (témoin, sujet sain) et/ou de réponse à un traitement médical (chirurgie, médicaments, radiothérapies,...).

On pourra suivre ainsi, en mesurant ce ratio (Grewis) à différents temps t1, t2, t3... au cours de la vie dudit sujet d'intérêt, les variations de ce ratio, indiquant une augmentation ou une diminution de la quantité d'ADN circulant chez ledit sujet. On parlera de variations longitudinales (dans le temps) de la quantité d'ADN circulant chez ledit sujet d'intérêt.

Un autre mode de réalisation utile à la compréhension de l'invention concerne donc également un procédé *in vitro* de diagnostic, pronostic, théranostic, ou de suivi de l'évolution d'un état physiologique spécifique d'un sujet d'intérêt susceptible de libérer de l'ADN circulant, en particulier un sujet atteint ou susceptible de développer un cancer ou une condition clinique choisie notamment parmi un AVC ou un infarctus myocardique, une insuffisance rénale aiguë, une cytolyse hépatique, un traumatisme, une chirurgie, un rejet de greffe, ou un sujet soumis à un exercice musculaire intensif, ou un sujet soumis à un traitement médical tel que biopsie, chirurgie, radiothérapie, chimiothérapie, immunothérapie, ou principe actif (chimique ou biologique) comprenant les étapes suivantes :
(i) à un temps t0, détection et/ou de quantification d'ADN libre de toutes cellules à partir d'un échantillon de fluide biologique d'un sujet d'intérêt selon le procédé de l'invention tel que sus-décrit;
(ii) à un temps t1, reproduction de l'étape (i), et
(iii) comparaison du ratio (Grewis) nombre de copies d'ADN circulant sur nombre de copies de fragment d'ADN exogène de 50-2000 paires de bases, de préférence 50-200 paires de bases selon l'invention, de préférence 60-160 paires de bases, de préférence encore 70-150 paires de bases et mieux de 80-140 paires de base (ICE) obtenu au temps t1 par rapport au même ratio obtenu au temps t0.

Les étapes (ii) et (iii) pouvant être reproduites autant de fois que nécessaire (par exemple à t2, t3, t4...) pour suivre l'évolution d'un état physiologique spécifique d'un sujet d'intérêt, et par voie de conséquence l'effet d'un traitement médical (chirurgie, médicaments, radiothérapies,...) sur ledit sujet.

Alternativement, un autre mode de réalisation utile à la compréhension de l'invention porte sur un procédé *in vitro* de diagnostic, pronostic, théranostic d'un sujet d'intérêt susceptible de libérer de l'ADN circulant, en particulier un sujet atteint ou susceptible de développer un cancer ou une condition clinique choisie notamment parmi un AVC ou un infarctus myocardique, une insuffisance rénale aiguë, une cytolyse hépatique, un traumatisme, une chirurgie, un rejet de greffe, ou un sujet soumis à un exercice musculaire intensif, ou un sujet soumis à un traitement médical tel que biopsie, chirurgie, radiothérapie, chimiothérapie, immunothérapie, ou principe actif (chimique ou biologique) comprenant au moins les étapes suivantes :
(i) à un temps t, détection et/ou de quantification d'ADN libre de toutes cellules à partir d'un échantillon de fluide biologique d'un sujet d'intérêt selon le procédé de détection et/ou quantification d'ADNcf selon l'invention ; et
(ii) comparaison du ratio (Grewis) du nombre de copies d'ADN circulant sur nombre de copies de fragment d'ADN exogène de 50-2000 paires de bases, de préférence 50-200 paires de bases selon l'invention, de préférence 60-160 paires de bases, de préférence encore 70-150 paires de bases et mieux de 80-140 paires de bases (ICE) obtenu au temps t par rapport au même ratio obtenu chez un sujet de référence (sujet sain, témoin).

La description va maintenant illustrer quelques applications du procédé selon l'invention.

### Applications dans le domaine de l'oncologie

Des progrès thérapeutiques majeurs ont été réalisés au cours des dernières années avec le développement des inhibiteurs de MAP-kinases (Tyrosine kinase inhibitors : TKIs) et des inhibiteurs de « check-point » immunitaires (anti-CTLA4 et anti-PD1 ; Immunothérapies). Compte tenu du coût et de la toxicité de ces molécules, et de la complexification des schémas thérapeutiques, il est de plus en plus essentiel de pouvoir bénéficier de biomarqueurs permettant de guider les choix thérapeutiques afin d'optimiser la prise en charge et de limiter les toxicités. Plusieurs équipes ont démontré il y a moins de 10 ans, des taux élevés d'ADN circulant chez des patients atteints de cancers gastriques, de l'ovaire ou de la prostate. En raison de la relative facilité d'accès et la nature de l'échantillon, l'utilisation de l'ADNcf est depuis quelques années pressentie pour devenir un outil d'aide au suivi de la progression ou de la réponse des patients aux traitements anticancéreux (Aung, K. L et al., Hugo J ; 2010). A ce jour, l'ADNcf peut ainsi être utilisé en recherche clinique, pour réaliser l'analyse des mutations somatiques de gènes d'intérêt thérapeutique dans certaines pathologies telles que : des cancers bronchiques non à petites cellules, des cancers du côlon, dans les cancers de la thyroïde et les mélanomes. Mais en date d'aujourd'hui, seule la prescription d'Osimertinib (Astra Zeneca) est autorisée dans le traitement des patients adultes atteints d'un cancer bronchique non à petites cellules (CBNPC) localement avancé ou métastatique, avec mutation EGFR T790M. L'AMM précise que le statut mutationnel EGFR T790M est déterminé par une méthode d'analyse validée en utilisant l'ADN tumoral circulant (ADNtc) obtenu à partir d'un échantillon de plasma. De nombreuses méthodes analytiques (ciblées ou non ciblées) permettent de rechercher des anomalies géniques à partir de l'ADNcf. Il est ainsi possible de détecter des Variations en Nombre (CNA), des mutations ponctuelles, insertions, délétions, insertions/délétions, méthylations du génome par méthodes ciblées : QPCR, pyroséquençage, High Resolution Melting curve (HRM), Mass-array, snap shot, dPCR... ou techniques non ciblées: CGH-array, SNP-array, séquençage sanger, NGS,.... Ces analyses permettent de pronostiquer, diagnostiquer ou encore prédire la réponse à un traitement curatif. En dehors des problèmes de sensibilité qui ne sont pas inhérentes aux seules performances analytiques mais aussi à la concentration en ADNcf et de la mutation, il n'est pas possible à ce jour de rendre un résultat d'analyse typé « normal » car il n'y a pas de méthode en routine clinique qui permettent de s'assurer de la présence d'ADN tumoral. Dans ce cas le résultat est rendu « non contributif» et une nouvelle biopsie solide ou liquide est nécessaire. De l'ADNcf peut être détecté chez les témoins sains et les sujets atteints d'affections non malignes, les traumatismes (Laktionov, P. P. et al., 2004.), procédures thérapeutiques (Davis, G. L. et al., Arthritis Rheum, 1973) et pratique du sport (Velders M. et al., Clinical Biochemistry, 2014) peuvent aussi entraîner la libération d'ADN dans la circulation. De ce fait, l'identification de marqueurs génétiques spécifiques au cancer est une garantie de suivre exclusivement l'ADN tumoral (Luke, J. J. et al., J Natl Cancer Inst, 2014). L'approche utilisée par certains laboratoires pour s'assurer de l'analyse d'ADNcf tumoral consiste à rechercher des anomalies génomiques individuelles somatiques comme marqueurs de la pathologie tumorale. Si l'on prend l'exemple du mélanome, le variant BRAF V600E peut ainsi permettre le monitoring des traitements par inhibiteurs spécifique de BRAF (Sanmamed, M. F. et al., Clin Chem, 2015). Dans ce cas, le choix de la cible est évident, mais ne permet pas une approche globale de la pathologie puisque seul environ 50% des patients sont mutés V600 et que le variant suivi est la cible du traitement et donc soumis à la pression du médicament. D'autres mutations plus rares peuvent également être analysées. Cette approche, est néanmoins complexe, coûteuse et difficile à réaliser en diagnostic de routine car elle doit être adaptée au profil moléculaire de chaque patient, ce qui signifie réaliser des analyses NGS de type exome. Ainsi, aucune technique pré-ou post-analytique ne permet de s'assurer de la présence de matériel contributif, et donc, pour chaque nouvelle biopsie liquide une analyse est réalisée en dehors de tout critère sélectif.

Le procédé de l'invention trouve ainsi dans le domaine de l'oncologie trois applications :

### 1- le suivi de la réponse à un traitement par mesure de la fluctuation d'ADNcf total au cours du temps:

L'analyse des fluctuations d'ADNcf pour suivre la réponse à un traitement (chirurgie, radiothérapie, chimiothérapies et autres principes actifs dont les thérapies ciblées incluant les immunothérapies) par la quantification d'ADN circulant libéré par la mort/lyse cellulaire de la tumeur, de ses métastases ou à leur périphérie. La technique d'extraction selon l'invention, IDXtract (ID-Solutions, France) permet de s'affranchir des contraintes préalablement énoncées, grâce à un l'ajout dans le plasma à extraire d'ADN exogène calibré : ICE (séquence non-humaine). Le fragment ICE subit les étapes d'extractions et permet de s'assurer de l'efficacité de l'extraction. Le kit selon l'invention de QPCR/dPCR IDQuant (ID-Solutions, France) permet de co-amplifier l'ADN exogène ICE avec une ou plusieurs séquences de génome humain. Il est possible de déterminer la quantité d'ADNcf et de ICE pour une même prise d'essai et de calculer la valeur du ratio ADNcf/ICE et de l'exprimer par ml de plasma (ADNcf/ICE/ml de plasma = Grewis). La variation du Grewis au cours du temps pour un même sujet d'intérêt permet de s'assurer de suivre longitudinalement les fluctuations en ADNcf d'origine physiologique/pathologique sans artefact.

### 2- le suivi d'une mutation :

La détection/quantification d'anomalies génomiques concomitantes à la mesure du Grewis et/ou de la séquence exogène ICE seul, permet de suivre l'évolution de la concentration en ADN muté entre les différentes biopsies liquides réalisées longitudinalement. Les résultats sont normalisés par rapport au fragment ICE (rajouté au plasma avant extraction de l'ADNcf) calibré par dPCR, exprimé par ml de plasma et/ou Grewis. Les variations en mutant permettent, par exemple, de suivre l'augmentation au cours du temps de séquence de l'ADNcf muté lié de manière directe ou indirecte à l'émergence de clones cellulaires tumoraux résistants au traitement ciblé ou non-ciblé, en cours. Il est par exemple possible de définir une valeur seuil décisionnelle de changement de ligne thérapeutique.

### 3- le rôle de `hub' analytique avant analyse génomique:

L'analyse d'anomalies génomiques somatiques à partir de tissus n'est réalisée qu'après s'être assuré de la présence de cellules tumorales dans le prélèvement. Comme expliqué au préalable, dans le cas des biopsies liquides et particulièrement le plasma, aucune méthode ne permet de s'assurer de la présence d'ADN circulant tumoral dans le prélèvement d'ADN libre total (ADNcf). Aucune méthode ne permet de s'assurer que les résultats seront contributifs avant de procéder à l'analyse génomique. L'utilisation des variations du Grewis selon l'invention permet de jouer le rôle de « hub » analytique :
- Diminution ou aucune variation du Grewis : pas d'analyse génomique requise ;
- Augmentation du Grewis : analyse génomique recommandée.

### Applications dans le domaine de la transplantation

Bien que les succès à court terme, après une transplantation d'organe solide, soient probants, l'incidence de rejet aigu varie entre les types de greffe. On observe des rejets aigus pour les transplantations intestinale, cardiaque, et pulmonaire (environ 55%, 30- 45% et de 35 à 40%, respectivement). Pour le foie, les reins-pancréas simultanés, et la transplantation rénale aiguë, le rejet est moins fréquent avec des incidences, 2 ans après la transplantation, de 4-6%, de 13-30%, 20%, et 12-14%, respectivement.

Ainsi, même pour la greffe à long terme, la survie du receveur reste peu satisfaisante. Malgré la prescription de médicaments immunosuppresseurs (ISDS), une immunosuppression excessive ou insuffisante peut se produire. Dans le cas des transplantations du pancréas, du rein et du foie, la mesure de marqueurs sanguins de type : lipase/amylase, créatinine sérique et enzymes hépatiques, respectivement, est recommandée pour la surveillance de routine post-transplantation. Toutefois, dans le domaine de la transplantation rénale, la détérioration de la fonction du greffon ne peut être détectée jusqu'à ce qu'un dommage significatif ne se produise. Après la transplantation cardiaque, la surveillance des enzymes cardiaques n'est pas recommandée en raison de la faible sensibilité de ces marqueurs dans le diagnostic de rejet aigu.

La détection non invasive des rejets apparaît encore plus intéressante dans le cas des rejets infra cliniques qui ne sont détectables actuellement qu'à partir de biopsies. À l'heure actuelle, AlloMap^{®} est le seul test de surveillance non invasif des transplantations d'organes dans le commerce. Il s'agit d'un test RT-QPCR de profil d'expression de 11 gènes, à partir des cellules mononucléaires du sang périphérique (PBMC), validé chez les transplantés cardiaques. Il est utilisé en suivi de transplantation cardiaque pour détecter un rejet aigu chez les patients entre 6 mois et 5 ans après la transplantation. Dans le rein, le même type d'approche est en cours de validation.

Depuis la découverte d'ADN acellulaire (ADNcf) dérivé du donneur (ddADNcf) dans le sang et l'urine du receveur, l'intérêt clinique de l'ADNcf dans le domaine de la transplantation est en plein développement.

Aujourd'hui, la transplantation pulmonaire est une thérapie établie pour les patients souffrant de maladies pulmonaires à un stade avancé. Bien que la survie après transplantation pulmonaire se soit statistiquement améliorée au cours de la dernière décennie, il existe toujours des complications telles que :
- Les complications liées à l'ischémie-reperfusion pendant la procédure de transplantation, définies comme dysfonctionnement primaire du greffon (PGD). Cette complication est la cause principale de décès dans le premier mois post-transplantation pulmonaire (TP) ;
- Le rejet aigu, qui se produit chez 33% des receveurs au cours de la première année post-transplantation. Les receveurs jeunes ont un taux de rejet aigu plus important, autour de 36% ;
- Les complications infectieuses liées à la fois à l'immunosuppression ainsi qu'à l'interface directe poumon-environnement externe ;
- Le rejet chronique qui concerne 49% et 76% des receveurs après 5 et 10 ans, respectivement.

Les premiers stades du rejet sont diagnostiqués par biopsie pulmonaire transbronchique après bronchoscopie qui est très sensible et spécifique. Cependant, cette technique est coûteuse, invasive et a une valeur prédictive limitée. La détection non invasive des rejets apparaît donc intéressante et notamment dans le cas des rejets infra cliniques qui ne sont détectables actuellement qu'à partir de biopsies.

L'identification de marqueurs non invasifs prédictifs des complications chez le transplanté pulmonaire est devenu aujourd'hui un véritable challenge.

En comparaison aux signatures moléculaires d'expression génique prédictives de rejet spécifiques d'organes, la quantification de l'ADNcf du donneur peut être un marqueur universel de tout type de transplantation d'organe solide. L'augmentation persistante ou un taux élevé d'ADN circulant peut signer un rejet aigu confirmé par biopsie chez les patients transplantés rénaux, pancréatiques ou cardiaques. Concernant la transplantation hépatique, le taux de l'ADNcf augmente dans le cas de rejet mais aussi dans les cas de stress, d'inflammation et avec le nombre de transfusion.

En 2015, De Vlaminck et al. ont conduit une étude prospective sur 51 patients (398 échantillons) transplantés pulmonaire. Le taux d'ADN circulant du donneur est estimé chez le receveur par la technique d'amplification en temps réel des polymorphismes SNP. La quantification de l'ADNcf du donneur était significativement plus élevée dans le groupe « Rejet cellulaire aigu modéré » ou « sévère » par rapport au groupe « stable ». En 2017, Zou et al. ont confirmé ces données chez 18 transplantés de poumon en détectant l'ADNcf du donneur par amplification des polymorphismes HLA par digital PCR. Malgré tous ces résultats prometteurs l'analyse en routine n'est pas encore effective car la variabilité artéfactuelle induite par les processus pré-analytiques sont négligés, en particuliers les étapes d'extraction de l'ADNcf ou les coefficients de variation sont proche de 20%.

En dehors du poumon, toutes les transplantations peuvent faire l'objet d'un suivi du rejet : cardiaque, rein, moelle osseuse, ...

Le procédé de l'invention permet avantageusement dans ce domaine de la transplantation de décliner deux applications :

### 1- suivre l'apparition d'un rejet de greffe :

L'analyse des fluctuations d'ADNcf pour détecter et suivre l'apparition d'un rejet de greffe par la quantification d'ADN circulant libéré par la mort/lyse cellulaire de l'organe ou de cellules transplantées (donneur). La technique d'extraction selon l'invention IDXtract (ID-Solutions, France) permet de s'affranchir des contraintes préalablement énoncées, grâce à un l'ajout dans le plasma à extraire d'ADN exogène calibrée : ICE (séquence non-humaine). Le fragment ICE subit les étapes d'extractions et permet de s'assurer de l'efficacité de l'extraction. Le kit selon l'invention de QPCR/dPCR IDQuant (ID-Solutions, France) permet de co-amplifier l'ADN exogène ICE avec une ou plusieurs séquences de génome humain. Il est possible de déterminer la quantité d'ADNcf et de ICE pour une même prise d'essai et de calculer la valeur du ratio ADNcf/ICE et de l'exprimer par ml de plasma (ADNcf/ICE/ml de plasma = Grewis). La variation du Grewis au cours du temps pour un même sujet d'intérêt permet de s'assurer de suivre longitudinalement les fluctuations en ADNcf d'origine physiologique/pathologique sans artefact. Ainsi la variation d'ADNcf total, détectée par PCR quantitative en temps réel ou digital PCR peut refléter un processus de rejet ;

### 2- suivre l'évolution de la concentration en ADN « donneur » :

La détection/quantification d'une signature en ADN du donneur à partir de ADNcf par ddPCR concomitante à la mesure du Grewis et/ou de la séquence exogène ICE permet de suivre l'évolution de la concentration en ADN « donneur » entre les différentes biopsies liquides réalisées longitudinalement. Les résultats sont normalisés par rapport au fragment ICE (rajouté au plasma avant extraction de l'ADNcf) calibré par dPCR, exprimé par ml de plasma et/ou Grewis. Les variations en séquence d'ADN donneur ou chimérisme permettent, par exemple, de suivre l'augmentation au cours du temps de ces séquences, qui peut être lié de manière directe ou indirecte à un rejet aigu ou infra-clinique. Il est par exemple possible :
- De détecter l'augmentation de la quantité d'ADN circulant du donneur qui peut signer un événement de rejet aigu quel que soit le type de transplantation d'organe.
- De définir une valeur seuil décisionnelle de changement de la pratique thérapeutique
- D'y voir un intérêt en tant que biomarqueurs de l'intégrité des organes (Kanzow P et al., Transplantation 2014) avec un suivi longitudinal standardisé.

### Diagnostic d'un état pathologique

En comparaison avec des valeurs moyennes de quantité d'ADNcf obtenues sur des sujets de référence (par exemple sujets sains : n=24 sujets, moyenne=9.38ng/ml plasma +/-4.47 ng/, mediane=8.94 ng/ml, min=3.21 ng/ml, max= 23.38 ng/ml), l'obtention de valeurs « anormales » du Grewis, c'est-à-dire des valeurs supérieures voire très supérieures aux valeurs des sujets de référence, signe un état pathologique, il est possible d'orienter le patient vers des consultations plus spécialisées pour affiner le diagnostic.

Un autre mode de réalisation utile à la compréhension de l'invention porte encore sur l'utilisation d'au moins un premier fragment d'ADN exogène de 50-2000 paires de bases, de préférence 50-200 paires de bases selon l'invention, de préférence 60-160 paires de bases, de préférence encore 70-150 paires de bases et mieux de 80-140 paires de bases (ICE), pour son utilisation dans la standardisation d'une méthode d'extraction et de quantification d'ADN libre de toutes cellules et/ou dans une méthode d'analyse des fluctuations d'ADN libre de toutes cellules chez un sujet d'intérêt dans le temps par mesure du ratio (Grewis) du nombre de copies d'ADN libre de toutes cellules sur le nombre de copies du premier fragment d'ADN exogène de 50-2000 paires de bases, de préférence 50-200 paires de bases selon l'invention, de préférence 60-160 paires de bases, de préférence encore 70-150 paires de bases et mieux de 80-140 paires de bases (ICE).

### KITS ET ENSEMBLES D'EXTRACTION, D'AMPLIFICATION-QUANTIFICATION DES ADNs CIRCULANTS

Un autre mode de réalisation utile à la compréhension de l'invention est un kit ou ensemble d'extraction d'ADN libre de toutes cellules à partir d'un échantillon de fluide biologique d'un sujet d'intérêt, comprenant :
(i) Au moins un premier fragment d'ADN exogène de 50-2000 paires de bases, de préférence 50-200 paires de bases selon l'invention, de préférence 60-160 paires de bases, de préférence encore 70-150 paires de bases et mieux de 80-140 paires de base (ICE);
(ii) Des tampons et enzymes, en particulier un tampon de protéolyse, une enzyme de protéolyse, un tampon de fixation, un tampon de lavage, et un tampon d'élution de l'ADN libre de toutes cellules,
(iii) Des dispositifs permettant l'isolation de l'ADN libre de toutes cellules, en particulier des colonnes d'extractions à membrane de silice et avantageusement des colonnes d'extractions surmontées d'accessoires de charge, de préférence des entonnoirs,
(iv) Des plaques multi-puits, en particulier des plaques 24-puits DeepWell de 25 mL
(v) Des tubes à vis, de préférence de 1,5ml, et
(vi) Une notice d'utilisation,
ledit fragment d'ADN exogène ICE étant fourni dans ledit kit ou apporté séparément audit kit, formant un ensemble d'extraction.

Selon un mode de réalisation non couvert par le texte des revendications, le kit d'extraction d'ADN circulant comprend :
(i) Au moins un premier fragment d'ADN exogène non humain de 300 paires de base et éventuellement un second fragment d'ADN exogène non humain de 110 paires de bases, tous deux étant des séquences d'ADN codant pour le précurseur d'une protéase à serine pro-agrégante de *Cerastes cerastes*
(ii) Des tampons et enzymes: un tampon de protéolyse avantageusement supplémenté en albumine sérique bovine (BSA) et/ou Gélatine de porc purifiée (Prionex^{®}), une enzyme de lyse, de préférence la protéinase K, un tampon de fixation, un tampon de lavage, un tampon d'élution,
(iii) Des colonnes d'extractions à membrane de silice, des plaques 24-puits DeepWell de 25 mL, des entonnoirs, des tubes à vise de 1,5 mL et
(iv) Une notice d'utilisation.

Selon un mode particulier non couvert par le texte des revendications, le kit d'extraction d'ADN circulant selon l'invention comprend :
(i) Au moins un premier fragment d'ADN exogène (ICE) non humain de 110 paires de bases, codant pour le précurseur d'une protéase à serine pro-agrégante de *Cerastes cerastes*
(ii) Des tampons et enzymes: un tampon de protéolyse avantageusement supplémenté en albumine sérique bovine (BSA) et/ou Gélatine de porc purifiée (Prionex^{®}), une enzyme de lyse, de préférence la protéinase K, un tampon de fixation, un tampon de lavage, un tampon d'élution,
(iii) Des colonnes d'extractions à membrane de silice, des plaques 24-puits DeepWell de 25 mL, des entonnoirs, des tubes à vise de 1,5 mL et
(iv) Une notice d'utilisation.

Selon un mode particulier non couvert par le texte des revendications, un kit pourra être caractérisé par les paramètres définis dans le tableau 1 suivant :

**Tableau 1**

| Technologie | Technologie de membrane de silice |
|---|---|
| Format | Colonnes Midi avec entonnoir amovible |
| Echantillon | 1 à 10ml de plasma, en particulier 1 à 5ml de plasma |
| Tubes de prélèvement sanguin | EDTA, Cell-Free DNA BCT^{®} (Streck) , Roche |
| Taille de fragments | ≥ 50 bp |
| Rendement | De 1 à 1000 ng |
| Volume d'élution avant concentration | 100-200 µl |
| Volume d'élution après concentration | 50 µl |
| Temps de préparation | 2h15 minutes/ 24préparations |

Selon un mode particulier non couvert par le texte des revendications, il s'agira d'un kit d'extraction d'ADN libre de toutes cellules à partir de plasma sanguin.

Ce kit permet la purification d'ADN libre de toutes cellules à partir d'un volume allant jusqu'à 100 ml de plasma, notamment 10ml de plasma, en particulier 5ml de plasma.

Le kit contient des colonnes Midi, de préférence avec des accessoires de charge desdites colonnes, de préférence des entonnoirs permettant de charger en une seule fois la totalité du mélange échantillon/solution d'extraction et permet le traitement de 1 à 24 échantillons en parallèle.

La fixation, le lavage, le séchage et l'élution des ADN libres de toutes cellules sont faits avec un dispositif de chambre à vide, et des support de colonnes (Starter S et Midi )

L'extraction se fait avantageusement sans ADN ou ARN entrainant (formation d'un complexe avec l'ADN afin de facilité son isolement). En effet, ces ADN ou ARN entrainant induisent la surestimation des concentrations d'ADN libres de toutes cellules lorsque l'on utilise des techniques de quantification d'ADN total, double ou simple brin.

Selon un mode particulier, le kit d'extraction d'ADN libre de toutes cellules selon l'invention est utilisé en association avec un kit d'amplification et de quantification d'ADN libre de toutes cellules, fournis ensemble ou séparément.

Selon un premier mode de réalisation, le kit d'extraction d'ADN libre de toutes cellules selon l'invention et le kit d'amplification et de quantification d'ADN libre de toutes cellules sont conditionnés ensemble.

Selon un premier mode de réalisation, le kit d'extraction d'ADN libre de toutes cellules selon l'invention et le kit d'amplification et de quantification d'ADN libre de toutes cellules sont conditionnés séparément.

Le kit d'amplification et de quantification d'ADN libre de toutes cellules comprendra généralement au moins :
(i) des amorces sens et antisens spécifiques respectivement de l'ADN libre de toutes cellules du sujet d'intérêt et du ou des fragments d'ADN exogènes tels que définis dans l'invention, pour l'étape d'amplification,
(ii) des sondes marquées respectivement pour l'ADN libre de toutes cellules du sujet d'intérêt et pour le ou les fragments d'ADN exogènes tels que définis dans l'invention pour l'étape de détection et quantification,
(iii) des tampons de réaction,
(iv) des plaques multi-puits et
(v) une notice d'utilisation.

### FIGURES

Figure 1 : représentation graphique de la variabilité des ADN circulants après extraction et de la normalisation de l'extraction et de la quantification grâce à l'utilisation d'un fragment exogène ICE selon le procédé de l'invention ;
Figure 2 : représentation graphique de l'analyse concomitante d'anomalies génomiques (mutations EGFR) et de la séquence exogène ICE et/ou du Grewis ;
Figure 3 : représentation graphique de l'analyse concomitante d'anomalies génomiques (mutations EGFR) et du Grewis.

La présente invention sera détaillée dans les exemples illustratifs et non limitatifs suivants.

### EXEMPLES

Les échantillons de plasmas sont préparés selon le protocole suivant :
- On prépare les plasmas à partir de sang total collecté sur tube BCT (streck) ou Cell-Free DNA collection tube (Roche) ou EDTA. Pour les tubes EDTA, le plasma doit être préparé dans les 4 heures qui suivent la prise de sang ;
- On centrifuge les tubes à 1600 x g pendant 10 minutes à température ambiante ;
- On collecte le surnageant (correspondant au plasma) puis on transfère dans un tube conique de 15 ml ;
- On centrifuge le plasma récupéré à 4500 g pendant 10 minutes à température ambiante ;
- On transfère le plasma dans un tube conique et mesure le volume ;
- On ajuster le volume de plasma au millilitre supérieur si nécessaire avec du tampon phosphate puis on le transfère dans un tube de 50 ml ou on le place dans une DeepWell de 25 ml.

De préférence, les échantillons de plasma sont préparés à partir d'un sujet d'intérêt susceptible de libérer des ADNcf, c'est-à-dire un sujet atteint ou susceptible de développer un cancer ou une condition clinique choisie notamment parmi un AVC ou un infarctus myocardique, une insuffisance rénale aiguë, une cytolyse hépatique, un traumatisme, une chirurgie, un rejet de greffe, ou un sujet soumis à un exercice musculaire intensif, ou un sujet soumis à un traitement médical tel que biopsie, chirurgie, radiothérapie, chimiothérapie, immunothérapie, ou principe actif (chimique ou biologique).

### Exemple 1 : Extraction standardisée d'ADN circulant à partir d'un échantillon de plasma

### 1.1 Protéolyse des plasmas

La protéolyse des échantillons de plasma tels que préparés ci-dessus est réalisée selon le protocole suivant :
- En plus des échantillons de plasma, un échantillon témoin CQI (lyophilisat de milieu de culture de lignée cellulaire de poumon humain référencée NCI-H1975, CRL-5908) autrement nommé contrôle de qualité d'extraction selon l'invention est utilisé pour contrôler la qualité d'extraction ;
- A chaque échantillon de plasma et échantillon témoin, on ajoute 30 µl de protéinase K liquide par ml d'échantillon ;
- Selon un mode de réalisation non couvert par le texte des revendications, on ajoute ensuite dans chaque échantillon par ml (plasma et échantillon témoin) 20000 copies du premier fragment d'ADN exogène de 300 paires de bases (ICE) représenté par la SEQ ID N°1 décrite ci-dessous et 20000 copies du second fragment d'ADN exogène de 110 paires de bases (LED) représenté par la SEQ ID N°2 décrite ci-dessous, tous deux étant des séquences d'ADN codant pour le précurseur d'une protéase à serine pro-agrégante de *Cerastes* cérastes ;
- On mélange au vortex brièvement puis on incube 5 minutes à température ambiante ;
- On ajoute ensuite dans chaque échantillon 400 µl de tampon de lyse (LYS) par ml de plasma ou de témoin;
- On mélange au vortex brièvement et on incube à 56°C pendant 30 minutes sous agitation ou 1 h pour les tubes Streck.

SEQ ID N°1 : 
CCTAATGACACTTATCCCAAAGTCCCTCATTGTGCTAACATTAACATACTTGAGCATTCGC
TGTGTGAAAGAGCTTACAATGATCTTTCGGCGAGTAGCAGAACATTGTGTGCAGGTATC
GAAAAAGGAGGCATAGATACATGTAAGGGTGACTCTGGGGGACCCCTCATCTGTAATGG
ACAAATCCAGGGCATTGTATCTTGGGGAGATGAAGTTTGTGGTAAACCTAATAAGCCTG
GCGTCTATACCAAGGTCTTTGATTATACTGACTGGATCCGGAACATTATTGCAGGAAATA
CA
SEQ ID N° 2 :

GCTGAACAAACCAGTTAACAACAGTACACACATCGCGCCTCTCAGCTTGCCTTCCAGTC
CTCCCAGTGTGGGCTCAGATTGCCGTATTATGGGATGGGGCACAATCACAT

### 1.2 Isolation de l'ADN circulant sur les colonnes :

L'isolation et la fixation de l'ADN circulant sur les colonnes est réalisée selon le protocole suivant :
- Pendant l'incubation de la lyse, on applique 1 ml de tampon d'activation des membranes de silice (ACTIVB) dans chaque colonne MIDI, on incube 1 minute puis on applique le vide à -0.4 bar pendant 1 minute ;
- On ajoute 2 ml de tampon de fixation (BB) par ml de plasma dans chaque échantillon lysé ;
- On mélange le contenu du tube par un minimum de 5 aspirations/refoulements ;
- On met en place les entonnoirs sur chaque colonne ;
- On applique le mélange échantillon lysé dans les colonnes MIDI ;
- Enfin, on applique le vide à -0.4 bar pendant 5 minutes en s'assurant que tout le lysat est bien passé à travers la colonne.

### 1.3 Lavage de la membrane :

L'étape de lavage de la membrane est réalisée selon le protocole suivant :
- On ajouter 4 ml de tampon de lavage de lavage Wash 1 dans chaque colonne, on incube 1 minute ;
- On applique le vide à -0.4 bar pendant 5 minutes ;
- On ajoute 2 ml de tampon de lavage Wash 2 dans chaque colonne, on incube 1 minute ;
- On applique le vide à -0.4 bar pendant 2 min ;
- On ajoute 2 ml de tampon Wash 2 dans chaque colonne, on incube 1 minute ;
- Enfin on applique le vide à -0.4 bar pendant 2 minutes.

### 1.4 Elution de l'ADN circulant

Avant de procéder à l'élution de l'ADN circulant, on appliquer le vide, au minimum -0.6 bar pendant 10 minutes pour sécher complètement la membrane de toute trace d'éthanol.

L'élution de l'ADN circulant est ensuite réalisée selon le protocole suivant :
- On ajoute ensuite 150 µl de tampon d'élution supplémenté extemporanément en Prionex à 1% (ELU) et préalablement chauffé à 70°C dans chaque colonne ;
- On incube 5 minutes à température ambiante ;
- On appliquer le vide à -0.5 bar pendant 30 secondes ;
- Enfin on applique à nouveau le vide à -0.5 bar pendant 10 sec

### 1.5 Concentration optionnelle des échantillons d'ADN circulant pour une analyse unique

La concentration de l'ADN circulant est réalisée selon le protocole suivant :
- On charge la totalité des éluats dans la plaque d'ultrafiltration ;
- On applique le vide à -0.5 bar 10 minutes (passage complet du volume) ;
- On ajoute 50 µl de tampon d'élution dans chaque puits contenant les échantillons ;
- On agite pendant 5 minutes à température ambiante ;
- Enfin on transfère les échantillons dans un nouveau tube préalablement labélisé.

### 1.6 Stockage et/ou conservation des ADN circulants extraits

Les ADN circulants obtenus doivent être conservés à 5°C ± 3°C si la ddPCR ou Q-PCR est réalisée en suivant, sinon les ADN circulants doivent être stockés à -80 °C pour une conservation plus longue.

Selon un mode de réalisation alternatif selon l'invention, on ajoute dans chaque échantillon par ml (plasma et échantillon témoin) entre 10000 et 20000 copies d'un premier fragment d'ADN exogène de 110 paires de bases (ICE) représenté par la SEQ ID N°2 codant pour le précurseur d'une protéase à serine pro-agrégante de *Cerastes cerastes* et jouant à la fois le rôle de ICE et LED.

### Exemple 2 : Détection et quantification d'ADN circulant avec contrôle interne (ICE)-fragment de 300 paires de bases (mode de réalisation non couvert par le texte des revendications)

La détection de l'ADN circulant extrait à l'exemple 1 et sa quantification est réalisée par droplet digital PCR (ddPCR) selon le protocole suivant, pour un volume réactionnel de 20 µl/échantillon :
- on met 10 µL de ddPCR Supermix pour sondes (no dUTP) (BioRad)
- on ajoute 0,1 µL d'une amorce sens dirigée contre le premier fragment d'ADN exogène pour le contrôle interne exogène ICE (TGG-ACA-AGG-ACA-TCA-TGC-TGA-T correspondant à la SEQ ID N°3) à 100 µM ;
- on ajoute 0,1 µL d'une amorce antisens dirigée contre le premier fragment d'ADN exogène pour le contrôle interne exogène ICE (GAC-TGG-AAG-GCA-AGC-TGA-GA correspondant à la SEQ ID N°4) à 100 µM ;
- on ajoute 0,06 µL de Sonde marquée HEX et BHQ1 dirigé contre le control interne exogène ICE (AAC-CAG-TTA-ACA-ACA-GTA-CAC-ACA-TCG-CGC correspondant à la SEQ ID N°5) à 100 µM ;
- on ajoute 0,1 µL d'une amorce sens dirigée contre le gène RPP-30 humain (GAT-TTG-GAC-CTG-CGA-GCG correspondant à la SEQ ID N°6) à 100 µM ;
- on ajoute 0,1 µL d'une amorce antisens dirigée contre le gène RPP-30 humain (GAG-CGG-CTG-TCT-CCA-CAA-GT correspondant à la SEQ ID N°7) à 100 µM ;
- on ajoute 0,06 µL de Sonde marquée FAM et MGB dirigé contre le gène RPP-30 humain (CTG-ACC-TGA-AGG-CTC-T correspondant à la SEQ ID N°8) à 100 µM ;
- on ajoute 1,48 µl de H₂O de qualité Biologie Moléculaire ;
- on ajoute 8 µL de l'ADN extrait (ADN circulant extrait et fragment d'ADN exogène);
- on homogénéise le mélange et on le charge dans un puit de DG8 cartridges (Biorad)
- on ajoute 70 µL d'huile pour la génération de gouttelettes dans la DG8 cartridges (Biorad)
- on met en place le joint DG8 gasket (Biorad) et on génère les gouttelettes en utilisant le Droplet Generator de Biorad ;
- on transfère l'échantillon dans une plaque 96-puits, on la scelle et on réalise une PCR temps-fini selon le programme ci-dessous :
   Etape 1 : 95°C 10 min
   Etape 2: 94°C 30 sec
   Etape 3 : 60°C 1 min
   Répéter étapes 2 et 3 40 fois
   Etape 4 : 98°C 10 minutes
   Etape 5 : 12°C 1 sec

A la fin de la PCR, on transfère la plaque 96-puits dans le lecteur de gouttelette Biorad et on effectue la lecture des gouttelettes.

On réalise la même étape d'amplification et quantification pour les ADNs extraits de l'échantillon de plasma et pour les ADNs extraits de l'échantillon témoin.

Les résultats obtenus pour le gène RPP30 représentent le nombre de copies d'ADN circulant extrait. Les résultats obtenus pour le fragment d'ADN exogène extrait (ICE) représentent le nombre de copies de ICE extrait.

Le calcul du Grewis s'effectue en divisant la valeur obtenue pour RPP30 par la valeur obtenue pour ICE. Cette valeur sert de valeur de référence que l'on peut comparer entre chaque extraction pour un même sujet et ainsi déterminer la variation de la quantité d'ADN libre dudit sujet au cours du temps.

On obtient un ratio (Grewis) représentatif de la quantité d'ADN circulant dans l'échantillon du sujet d'intérêt.

### Exemple 3 : Détection et quantification d'ADN circulant avec en outre contrôle du profil de taille (LED)- fragment ICE de 300 paires de bases et fragment LED de 110 paires de bases (mode de réalisation non couvert par le texte des revendications)

La détection de l'ADN circulant extrait à l'exemple 1 et sa quantification est réalisée par droplet digital PCR (ddPCR) selon le protocole suivant, pour un volume réactionnel de 20 µl/échantillon :
- on met 10 µL de ddPCR Supermix pour sondes (no dUTP) (BioRad)
- on ajoute 0,1 µL d'une amorce sens dirigée contre le premier fragment d'ADN exogène pour le contrôle interne exogène ICE (TGG-ACA-AGG-ACA-TCA-TGC-TGA-T correspondant à la SEQ ID N°3) à 100 µM ;
- on ajoute 0,1 µL d'une amorce antisens dirigée contre le premier fragment d'ADN exogène pour le contrôle interne exogène ICE (GAC-TGG-AAG-GCA-AGC-TGA-GA correspondant à la SEQ ID N°4) à 100 µM ;
- on ajoute 0,06 µL de Sonde marquée HEX et BHQ1 dirigé contre le control interne exogène ICE (AAC-CAG-TTA-ACA-ACA-GTA-CAC-ACA-TCG-CGC correspondant à la SEQ ID N°5) à 100 µM ;
- on ajoute 0,1 µL d'une amorce sens dirigée contre le second fragment d'ADN exogène pour contrôle d'extraction des fragments de petite taille LED (ATG-ACA-CTT-ATC-CCA-AAG-TCC-CTC correspondant à la SEQ ID N°9) à 100 µM ;
- on ajoute 0,1 µL d'une amorce antisens dirigée contre le second fragment d'ADN exogène pour contrôle d'extraction des fragments de petite taille LED (CAA-TGT-TCT-GCT-ACT-CGC-CGA correspondant à la SEQ ID N°10) à 100 µM ;
- on ajoute 0,06 µL de Sonde marquée FAM et MGB dirigé contre le control interne exogène LED (CAT-ACT-TGA-GCA-TTC-GCT-GT correspondant à la SEQ ID N°11) à 100 µM ;
- on ajoute 1,48 µl de H₂O de qualité Biologie Moléculaire ;
- on ajoute 8 µL de l'ADN extrait (ADN circulant extrait et second fragment d'ADN exogène);
- on homogénéise le mélange et on le charge dans un puit de DG8 cartridges (Biorad)
- on ajoute 70 µL d'huile pour la génération de gouttelettes dans la DG8 cartridges (Biorad)
- on met en place le joint DG8 gasket (Biorad) et on génère les gouttelettes en utilisant le Droplet Generator de Biorad ;
- on transfère l'échantillon dans une plaque 96-puits, on la scelle et on réalise une PCR temps-fini selon le programme ci-dessous :
   Etape 1 : 95°C 10 min
   Etape 2: 94°C 30 sec
   Etape 3 : 60°C 1 min
   Répéter étapes 2 et 3 40 fois
   Etape 4 : 98°C 10 minutes
   Etape 5 : 12°C 1 sec

A la fin de la PCR, on transfère la plaque 96-puits dans le lecteur de gouttelette Biorad et on effectue la lecture des gouttelettes.

On réalise les mêmes étapes d'extraction, d'amplification et quantification pour les ADNs extraits de l'échantillon de plasma et pour les ADNs extraits de l'échantillon témoin.

Les résultats obtenus pour le fragment d'ADN exogène extrait (ICE) représentent le nombre de copies de ICE extrait. Les résultats obtenus pour le second fragment d'ADN exogène extrait (LED) représentent le nombre de copies de LED extrait.

Le calcul du contrôle d'extraction des fragments de petites tailles s'effectue en divisant la valeur obtenue pour ICE par la valeur obtenue pour LED.

On obtient des ratios ICE/LED proches de 1, qui doivent rester stables d'une extraction à l'autre. Si ce ratio augmente, cela signifie que les ADN de petites tailles n'ont pas été extraits correctement, et que les résultats obtenus sur la quantification de l'ADN circulant sont biaisés.

On peut également réaliser une amplification et quantification en utilisant simultanément les 3 cibles (ADN circulant, ICE et LED) telles que décrites respectivement dans les exemples 2 et 3 ci-dessus.

### Exemple 4 : Détection et quantification d'ADN circulant avec contrôle interne (ICE) de 110 paires de bases jouant également le rôle de LED (mode de réalisation selon l'invention revendiquée)

On reproduit l'exemple 2 ci-dessus en utilisant, comme fragment exogène ICE, le fragment de 110 paires de bases correspondant à la séquence SEQ ID N°2.

La détection de l'ADN circulant extrait à l'exemple 1 et sa quantification est réalisée par droplet digital PCR (ddPCR) selon le protocole suivant, pour un volume réactionnel de 20 µl/échantillon :
- on met 10 µL de ddPCR Supermix pour sondes (no dUTP) (BioRad)
- on ajoute 0,1 µL d'une amorce sens dirigée contre le fragment d'ADN exogène pour le contrôle interne exogène ICE (ATG-ACA-CTT-ATC-CCA-AAG-TCC-CTC correspondant à la SEQ ID N°9) à 100 µM ;
- on ajoute 0,1 µL d'une amorce antisens dirigée contre le fragment d'ADN exogène pour le contrôle interne exogène ICE (CAA-TGT-TCT-GCT-ACT-CGC-CGA correspondant à la SEQ ID N°10) à 100 µM ;
- on ajoute 0,06 µL de Sonde marquée FAM et MGB dirigé contre le control interne exogène ICE (CAT-ACT-TGA-GCA-TTC-GCT-GT correspondant à la SEQ ID N°11) à 100 µM ;
- on ajoute 0,1 µL d'une amorce sens dirigée contre le gène RPP-30 humain (GAT-TTG-GAC-CTG-CGA-GCG correspondant à la SEQ ID N°6) à 100 µM ;
- on ajoute 0,1 µL d'une amorce antisens dirigée contre le gène RPP-30 humain (GAG-CGG-CTG-TCT-CCA-CAA-GT correspondant à la SEQ ID N°7) à 100 µM ;
- on ajoute 0,06 µL de Sonde marquée FAM et MGB dirigé contre le gène RPP-30 humain (CTG-ACC-TGA-AGG-CTC-T correspondant à la SEQ ID N°8) à 100 µM ;
- on ajoute 1,48 µl de H₂O de qualité Biologie Moléculaire ;
- on ajoute 8 µL de l'ADN extrait (ADN circulant extrait et fragment d'ADN exogène);
- on homogénéise le mélange et on le charge dans un puit de DG8 cartridges (Biorad)
- on ajoute 70 µL d'huile pour la génération de gouttelettes dans la DG8 cartridges (Biorad)
- on met en place le joint DG8 gasket (Biorad) et on génère les gouttelettes en utilisant le Droplet Generator de Biorad ;
- on transfère l'échantillon dans une plaque 96-puits, on la scelle et on réalise une PCR temps-fini selon le programme ci-dessous :
   Etape 1 : 95°C 10 min
   Etape 2: 94°C 30 sec
   Etape 3 : 60°C 1 min
   Répéter étapes 2 et 3 40 fois
   Etape 4 : 98°C 10 minutes
   Etape 5 : 12°C 1 sec

A la fin de la PCR, on transfère la plaque 96-puits dans le lecteur de gouttelette Biorad et on effectue la lecture des gouttelettes.

On réalise la même étape d'amplification et quantification pour les ADNs extraits de l'échantillon de plasma et pour les ADNs extraits de l'échantillon témoin.

Les résultats obtenus pour le gène RPP30 représentent le nombre de copies d'ADN circulant extrait. Les résultats obtenus pour le fragment d'ADN exogène extrait (ICE) représentent le nombre de copies de ICE extrait.

Le calcul du Grewis s'effectue en divisant la valeur obtenue pour RPP30 par la valeur obtenue pour ICE. Cette valeur sert de valeur de référence que l'on peut comparer entre chaque extraction pour un même sujet et ainsi déterminer la variation de la quantité d'ADN libre dudit sujet au cours du temps.

On obtient un ratio (Grewis) représentatif de la quantité d'ADN circulant dans l'échantillon du sujet d'intérêt.

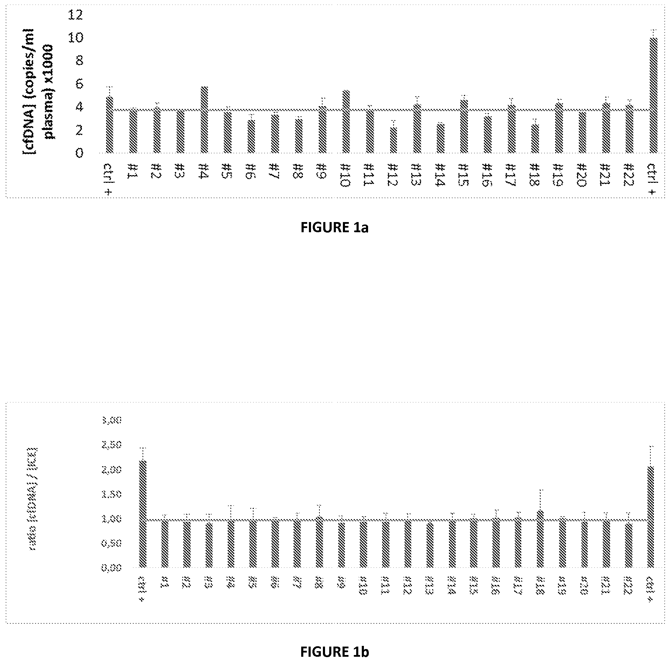

On observe à la Figure 1a une variabilité intra-essais des ADNcf après extraction, en l'absence de fragment d'ADN exogène ICE. En présence d'un fragment exogène ICE, calibré par ddPCR et rajouté au plasma avant extraction, on observe à la Figure 1b une homogénéité de la valeur du ratio ADN génomique/ICE (Grewis). Le fragment ICE permet de normaliser l'extraction et la quantification.

La variation du Grewis seul au cours du temps pour un même sujet d'intérêt permet de s'assurer de suivre longitudinalement les fluctuations en ADNcf d'origine physiologique/pathologique sans artefact.

### Exemple 5 : Procédé de suivi de l'état physiologique d'un sujet d'intérêt avec recherche concomitante de mutation EGFR dans les Cancers Bronchiques Non à Petites Cellules

On utilise, en association avec un kit d'extraction et de détection d'ADNcf tels que décrits dans les exemples 1 et 4, un kit IDEGFR de détection et de quantification par PCR digital des mutations de l'EGFR impliquées dans la stratification des patients pour les thérapies ciblées utilisant des inhibiteurs de tyrosine kinase et les résistances associées.

Il s'agit d'un système multiplex de quantification qui permet, pour chaque échantillon, l'amplification simultanée en 2 réactions des mutations activatrices (L858R et Del19- 1^{ère} réaction) et des mutations de résistance (T790M et C797S- 2^{ème} réaction). Les mutations activatrices du gène codant pour l'EGFR apparaissent principalement dans le cancer du poumon non à petites cellules (CPNPC) et provoquent l'activation constitutive de l'activité kinase de la protéine EGFR, contribuant ainsi au processus oncogénique. Les mutations les plus communes incluent une variété de délétions au niveau de l'exon 19 et une subsititution L858R dans l'exon 21. Ces mutations constituent 85% des mutations de l'EGFR observées dans les CPNPC. Les mutations T790M et C797S, représentent 2 mutations décrites comme conférant une résistance aux thérapies ciblées Erlotinib et Osimertinib respectivement.

Le kit IDEGFR contient les réactifs suivants :
- Un témoin positif cible (TPC-IDEGFR) comprenant un mélange d'ADN synthétique portant les différentes mutations et calibré à la concentration mentionnée dans la fiche de contrôle qualité associée ;
- Un mélange réactionnel d'amplification (ARM-IDEGFR sensi) contenant la Taq Polymérase et les oligonucléotides pour la détection de l'EGFR (FAM/HEX) et des mutations L858R (FAM) et délétion Exon 19 (HEX) ;
- Un mélange réactionnel d'amplification (ARM-IDEGFR resist) contenant la Taq polymérase et els oligonucléotides pour la détection de l'EGFR (FAM low) et des mutations T790M (FAM high) et C797S (HEX) ;
- Un témoin négatif d'extraction (NEC) préparé et extrait de la même manière que les échantillons à tester mais ne contenant pas d'échantillon (remplacé par du PBS) ;
- Un témoin négatif d'amplification (NAC) correspondant au dépôt de 13µl de mélange réactionnel d'amplification et 8µl d'eau Nucléase-free.

L'ADN circulant est extrait des échantillons selon le protocole décrit à l'exemple 4, avant d'être amplifié.

La réaction d'amplification par PCR temps fini (PCR digital QX200 Biorad) est réalisée dans chaque puits en présence de 13µl d'ARM-IDEGFR (Sensi ou Resist) pour chaque échantillon aisni que pour les contrôles positifs et négatifs. On ajoute dans chaque mix réactionnel :
- 8µl d'ADN extrait de chaque échantillon à analyser
- 8µl d'ADN TPC-IDEGFR
- 8µl d'extrait de NEC
- 8µl d'eau Nuclease-free (NAC).

La phase d'amplification est réalisée selon le programme suivant :
1) Activation de la polymérase (10min à 95°C, 1 cycle) ;
2) Dénaturation de l'ADN/élongation (15 secondes à 95°C et 60 secondes à 60°C, 40 cycles) ;
3) Consolidation des goutelettes (10 minutes à 98°C, 1 cycle) ;
4) Refroidissement (temps infini à 12°C, 1 cycle).

Les résultats sont analysés via le logiciel QuantaSoft^{™} Analysis Pro.

Une mutation est considérée positive si la valeur en goutelettes positives est supérieure ou égale à 2.

Une mutation est considérée non détectée si sa valeur est nulle ou inférieure à 2 goutelettes positives.

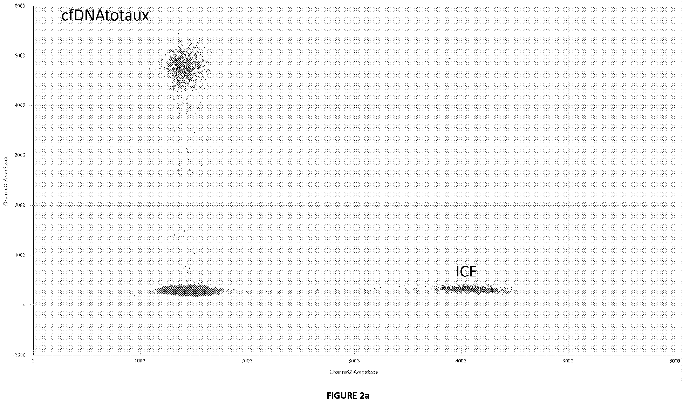

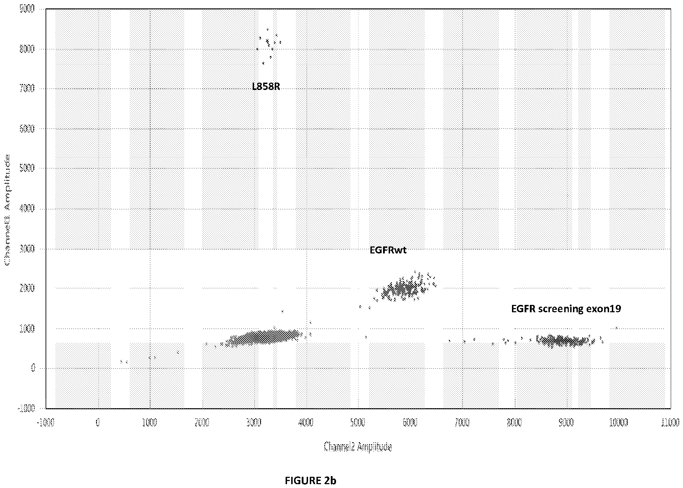

On observe à la Figure 2a les résultats de la co-amplification de l'ADNcf (RPP30) et ICE par dPCR. La Figure 2b représente l'analyse des mutations EGFR par le kit IDEGFR tube mutations *ARM-IDEGFR sensi.*

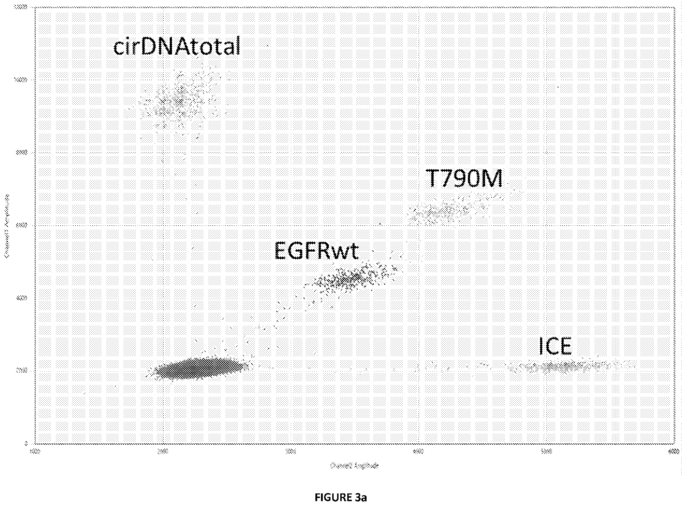

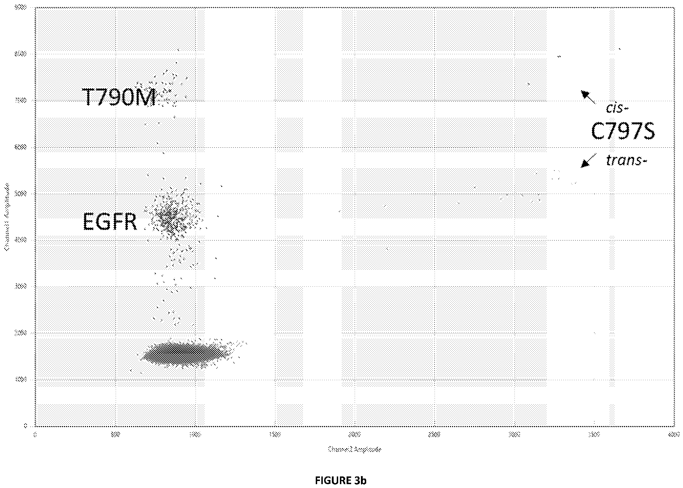

La figure 3a illustre l'analyse des mutations EGFR par le kit IDEGFR tube mutations ARM-*IDEGFR resist* avec co-amplification de ICE. Et la Figure 3b illustre l'analyse des mutations EGFR par le kit IDEGFR tube mutations *ARM-IDEGFR resist.*

La détection/quantification d'anomalies génomiques concomitante à la mesure du Grewis et/ou de la séquence exogène ICE permet de suivre l'évolution de la concentration en ADN muté entre les différentes biopsies liquides réalisées longitudinalement.

En fonction de la fluctuation du % de mutant normalisé par le Grewis, le traitement ciblé est adapté.

### Exemple 6 : Procédé de détection/quantification d'une signature en ADN du donneur à partir de ADNcf par ddPCR concomitante à la mesure du Grewis et/ou de la séquence exogène ICE

On utilise, en association avec un kit d'extraction et de détection d'ADNcf tels que décrits dans les exemples 1 et 4, un kit de détection et de quantification par PCR digital d'ADN donneur (après transplantation). Le protocole est similaire au protocole décrit à l'exemple 5 pour l'ADN muté, à la différence près que les sondes utilisées sont spécifiques de l'ADN donneur en remplacement de l'ADN muté.

La variation au cours du temps du Grewis est consécutive, dans un état pathologique, à une fluctuation de ADNcf physiologique/pathologique :
- Une augmentation du ratio signe un rejet de greffe ;
- Une diminution du ratio signe une prise du greffon ;
- Un état stationnaire signe que le greffon est intact.

La détection/quantification d'ADN 'donneur' concomitante à la mesure du Grewis et/ou de la séquence exogène ICE permet en outre de suivre l'évolution de la concentration en ADN « donneur » entre les différentes biopsies liquides réalisées longitudinalement.

### Exemple 7 : Utilisation de la mesure du Grewis et/ou de la séquence exogène ICE dans un process analytique d'analyse génomique (rôle de 'hub')

On utilise un kit d'extraction, détection et amplification d'ANDcf selon les protocoles décrits dans les exemples 1 et 4, en présence d'un fragment ICE.

La variation du Grewis consécutive, dans un état pathologique, à une fluctuation de ADNcf physiologique/pathologique, permet de décider s'il est opportun ou non de procéder à une analyse génomique subséquente :
- Pas de fluctuation ou augmentation du Grewis : analyse génomique inutile ;
- Si augmentation du Grewis : analyse génomique utile ; on concentre l'ADNcf à l'issue de l'étape d'extraction avant amplification ou toute autre analyse de génomique avec technique ciblée ou non ciblée de séquençage (NGS et autres).

### Exemple 8 : Procédé in vitro d'aide au diagnostic d'un état pathologique

En comparaison avec des valeurs moyennes obtenues sur des sujets sains et une persistance de valeurs « anormales » du grewis, il est possible d'orienter le sujet d'intérêt vers des consultations plus spécialisées pour affiner le diagnostic :
- Diminution du ratio (Grewis) : réponse à un traitement médical, quel qu'il soit (chirurgie, principe actif...), suggérant l'efficacité du traitement et donc son maintien ;
- Augmentation du ratio (Grewis) : réponse insuffisante ou non réponse à un traitement, suggérant un changement de traitement ;
- Etat stationnaire du ratio (Grewis): le traitement est toujours efficace.

## Revendications

1. Procédé de détection et/ou de quantification d'ADN libre de toutes cellules à partir d'un échantillon de fluide biologique d'un sujet d'intérêt distinct d'un sujet normal ou sain comprenant au moins :
(i) une étape d'extraction d'ADN libre de toutes cellules à partir d'un échantillon de fluide biologique dans lequel on a ajouté au moins une quantité efficace d'un premier fragment d'ADN exogène de 50-200 paires de bases, de préférence 60-160 paires de bases, de préférence encore 70-150 paires de bases et mieux de 80-140 paires de bases (ICE);
(ii) une étape de co-amplification et quantification de l'ADN libre de toutes cellules et du fragment d'ADN exogène ICE extrait à l'étape (i), et
(iii) une étape de standardisation de la quantité d'ADN libre de toutes cellules extrait comprenant le calcul d'un premier ratio, par ml d'échantillon de fluide biologique, du nombre de copies d'ADN libre de toutes cellules sur le nombre de copies du premier fragment d'ADN exogène de 50-200 paires de bases, de préférence 60-160 paires de bases, de préférence encore 70-150 paires de bases et mieux de 80-140 paires de bases (ICE),
**caractérisé en ce que** le sujet d'intérêt est atteint ou développe un cancer ou une condition pathologique choisie parmi un AVC ou un infarctus myocardique, une insuffisance rénale aiguë, une cytolyse hépatique, un traumatisme, un rejet de greffe ou un traitement médical tel que biopsie, chirurgie, radiothérapie, chimiothérapie, immunothérapie ou principe actif .

2. Procédé de détection et/ou de quantification d'ADN libre de toutes cellules à partir d'un échantillon de fluide biologique selon la revendication 1, **caractérisé en ce que** le sujet d'intérêt distinct d'un sujet normal ou sain est atteint ou développe un cancer.

3. Procédé selon la revendication 1 ou 2, **caractérisé en ce que** le fluide biologique est choisi dans le groupe constitué par le sang total, le sérum, le plasma, l'urine, la salive, l'effluent de la moelle osseuse, la lymphe, le liquide céphalorachidien, le liquide lacrymal, la sueur, le lait, l'humeur aqueuse, le liquide synovial, le liquide pleural, liquide péritonéal, liquide amniotique, la bile, liquide séminales, expectorations, de préférence le plasma.

4. Procédé de détection et/ou de quantification d'ADN libre de toutes cellules à partir d'un échantillon de fluide biologique selon l'une des revendications 1 à 3, **caractérisé en ce que** l'étape d'extraction (i) comprend les étapes suivantes :
(i1) Protéolyse dudit échantillon de fluide biologique d'un sujet d'intérêt distinct d'un sujet normal ou sain dans lequel on a ajouté au moins un premier fragment d'ADN exogène de 50-200 paires de bases, de préférence 60-160 paires de bases, de préférence encore 70-150 paires de bases et mieux de 80-140 paires de bases (ICE),
(i2) Isolation de l'ADN libre de toutes cellules, notamment par affinité sur un support de membrane, de préférence un support de membrane de silice dans une colonne d'extraction,
(i3) Elution de l'ADN libre de toutes cellules isolé à l'étape (i2) dans une phase aqueuse,
(i4) Eventuellement précipitation de l'ADN libre de toutes cellules,
(i5) Avantageusement concentration de l'ADN libre de toutes cellules,
(i6) Eventuellement conservation et/ou stockage dudit ADN libre de toutes cellules.

5. Procédé de détection et/ou de quantification d'ADN libre de toutes cellules à partir d'un échantillon de fluide biologique d'un sujet d'intérêt distinct d'un sujet normal ou sain selon l'une quelconque des revendications 1 à 4, **caractérisé en ce qu'**il comprend une co-amplification, en particulier dans le même puits, d'un ADN libre de toutes cellules, d'un fragment d'ADN exogène (ICE) et d'une séquence d'ADN génomique choisie parmi une séquence d'ADN muté, ou une séquence d'ADN du donneur dans le cas particulier d'un sujet d'intérêt transplanté susceptible d'avoir un rejet de greffe.

6. Procédé de détection et/ou de quantification d'ADN libre de toutes cellules à partir d'un échantillon de fluide biologique d'un sujet d'intérêt distinct d'un sujet normal ou sain selon l'une quelconque des revendications 1 à 5, **caractérisé en ce que** l'étape (ii) d'amplification et quantification de l'ADN libre de toutes cellules extrait à l'étape précédente est réalisée par une méthode de digital PCR, Q-PCR et/ou NGS, en particulier la digital PCR.

7. Utilisation du procédé de détection et/ou de quantification d'ADN libre de toutes cellules à partir d'un échantillon de fluide biologique d'un sujet d'intérêt distinct d'un sujet normal ou sain selon l'une quelconque des revendications 1 à 6, dans une méthode d'analyse d'un échantillon biologique d'un sujet d'intérêt distinct d'un sujet normal ou sain susceptible de libérer des ADNs libres de toutes cellules, ledit sujet d'intérêt étant atteint ou développe un cancer ou une condition clinique pathologique choisie parmi un AVC ou un infarctus myocardique, une insuffisance rénale aiguë, une cytolyse hépatique, un traumatisme, un rejet de greffe, ou un sujet soumis à un traitement médical tel que biopsie, chirurgie, radiothérapie, chimiothérapie, immunothérapie, ou principe actif.

8. Procédé *in vitro* d'analyse, en particulier de diagnostic, pronostic, théranostic, ou de suivi de l'évolution d'un état physiologique spécifique d'un sujet d'intérêt distinct d'un sujet normal ou sain susceptible de libérer de l'ADN circulant, ledit sujet d'intérêt étant atteint ou développe un cancer ou une condition clinique pathologique choisie parmi un AVC ou un infarctus myocardique, une insuffisance rénale aiguë, une cytolyse hépatique, un traumatisme, un rejet de greffe, ou un sujet soumis à un traitement médical tel que biopsie, chirurgie, radiothérapie, chimiothérapie, immunothérapie, ou principe actif., comprenant les étapes suivantes :
(i) à un temps t0, détection et/ou de quantification d'ADN libre de toutes cellules à partir d'un échantillon de fluide biologique d'un sujet d'intérêt distinct d'un sujet normal ou sain selon l'une quelconque des revendications 1 à 6;
(ii) à un temps t1, reproduction de l'étape (i), et
(iii) comparaison du ratio, par ml d'échantillon de fluide biologique, du nombre de copies d'ADN circulant sur nombre de copies de fragment d'ADN exogène de 50-200 paires de bases, de préférence 60-160 paires de bases, de préférence encore 70-150 paires de bases et mieux de 80-140 paires de bases (ICE) obtenu au temps t1 par rapport au même ratio obtenu au temps t0.

9. Procédé *in vitro* d'analyse selon la revendication 8, destiné notamment à :
- analyser les fluctuations d'ADN libre de toutes cellules pour suivre la réponse et/ou résistance à un traitement médical tel que biopsie, chirurgie, radiothérapie, chimiothérapie, immunothérapie, ou principe actif, ou
- analyser les fluctuations d'ADN libre de toutes cellules pour suivre l'apparition d'un rejet de greffe, ou
- analyser les fluctuations d'ADN libre de toutes cellules pour décider de l'intérêt de procéder à une analyse subséquente d'anomalies génomiques, ou
- suivre l'évolution de la concentration en ADN muté, ou
- suivre l'évolution de la concentration en ADN du donneur dans le cas particulier d'un sujet d'intérêt transplanté susceptible d'avoir un rejet de greffe.

10. Procédé *in vitro* de diagnostic, pronostic, théranostic d'un sujet d'intérêt distinct d'un sujet normal ou sain susceptible de libérer de l'ADN circulant, ledit sujet d'intérêt étant atteint ou développe un cancer ou une condition clinique pathologique choisie parmi un AVC ou un infarctus myocardique, une insuffisance rénale aiguë, une cytolyse hépatique, un traumatisme, un rejet de greffe, ou un sujet soumis à un traitement médical tel que biopsie, chirurgie, radiothérapie, chimiothérapie, immunothérapie, ou principe actif, comprenant les étapes suivantes :
(i) à un temps t, détection et/ou de quantification d'ADN libre de toutes cellules à partir d'un échantillon de fluide biologique d'un sujet d'intérêt distinct d'un sujet normal ou sain selon l'une quelconque des revendications 1 à 6;
(ii) comparaison du ratio, par ml d'échantillon de fluide biologique, du nombre de copies d'ADN circulant sur nombre de copies de fragment d'ADN exogène de 50-200 paires de bases, de préférence 60-160 paires de bases, de préférence encore 70-150 paires de bases et mieux de 80-140 paires de bases (ICE) obtenu au temps t par rapport au même ratio obtenu chez un sujet de référence (sujet sain, témoin).

11. Utilisation, dans un procédé de détection et/ou de quantification d'ADN libre de toutes cellules à partir d'un échantillon de fluide biologique d'un sujet d'intérêt distinct d'un sujet normal ou sain selon l'une quelconque des revendications 1 à 6 ou dans un procédé *in vitro* d'analyse selon la revendication 8 ou dans un procédé *in vitro* de diagnostic, pronostic, théranostic d'un sujet d'intérêt distinct d'un sujet normal ou sain selon la revendication 10, d'un kit ou ensemble d'extraction d'ADN libre de toutes cellules à partir d'un échantillon de fluide biologique d'un sujet d'intérêt distinct d'un sujet normal ou sain, ledit sujet d'intérêt étant atteint ou développe un cancer ou une condition clinique pathologique choisie parmi un AVC ou un infarctus myocardique, une insuffisance rénale aiguë, une cytolyse hépatique, un traumatisme, un rejet de greffe, ou un sujet soumis à un traitement médical tel que biopsie, chirurgie, radiothérapie, chimiothérapie, immunothérapie, ou principe actif comprenant :
(i) Au moins un premier fragment d'ADN exogène de 50-200 paires de bases, de préférence 60-160 paires de bases, de préférence encore 70-150 paires de bases et mieux de 80-140 paires de base (ICE);
(ii) Des tampons et enzymes, en particulier un tampon de protéolyse, une enzyme de protéolyse, un tampon de fixation, un tampon de lavage, et un tampon d'élution de l'ADN libre de toutes cellules,
(iii) Des dispositifs permettant l'isolation de l'ADN libre de toutes cellules, en particulier des colonnes d'extractions à membrane de silice et avantageusement des colonnes d'extractions surmontées d'accessoires de charge, de préférence des entonnoirs,
(iv) Des plaques multi-puits, en particulier des plaques 24-puits DeepWell de 25 mL
(v) Des tubes à vis, de préférence de 1,5ml, et
(vi) Une notice d'utilisation,
ledit fragment d'ADN exogène ICE étant fourni dans ledit kit ou apporté séparément audit kit, formant un ensemble d'extraction.

12. Utilisation selon la revendication 11 dans un procédé *in vitro* selon l'une quelconque des revendications 1 à 10, d'un kit ou ensemble d'extraction d'ADN libre de toutes cellules tel que défini dans la revendication 11, en association avec un kit d'amplification et de quantification d'ADN libre de toutes cellules, fournis ensemble ou séparément, ledit kit d'amplification et de quantification d'ADN libre de toutes cellules comprenant au moins :
(i) des amorces sens et antisens spécifiques respectivement de l'ADN libre de toutes cellules du sujet d'intérêt distinct d'un sujet normal ou sain et du fragment d'ADN exogène tels que définis dans la revendication 1,
(ii) des sondes marquées respectivement pour l'ADN libre de toutes cellules du sujet d'intérêt distinct d'un sujet normal ou sain et pour le fragment d'ADN exogène tels que définis dans la revendication 1,
(iii) des tampons de réaction
(iv) des plaques multi-puits et
(v) une notice d'utilisation.

13. Utilisation, dans un procédé de détection et/ou de quantification d'ADN libre de toutes cellules à partir d'un échantillon de fluide biologique d'un sujet d'intérêt distinct d'un sujet normal ou sain selon l'une quelconque des revendications 1 à 6 ou dans un procédé in vitro d'analyse selon la revendication 8 ou dans un procédé in vitro de diagnostic, pronostic, théranostic d'un sujet d'intérêt distinct d'un sujet normal ou sain selon la revendication 10, d'au moins un premier fragment d'ADN exogène de 50-200 paires de bases de préférence 60-160 paires de bases, de préférence encore 70-150 paires de bases et mieux de 80-140 paires de bases (ICE), permettant de standardiser une méthode d'extraction et de quantification d'ADN libre de toutes cellules et/ou d'analyser les fluctuations d'ADN libre de toutes cellules chez un sujet d'intérêt distinct d'un sujet normal ou sain, ledit sujet d'intérêt étant atteint ou développe un cancer ou une condition clinique pathologique choisie parmi un AVC ou un infarctus myocardique, une insuffisance rénale aiguë, une cytolyse hépatique, un traumatisme, un rejet de greffe, ou un sujet soumis à un traitement médical tel que biopsie, chirurgie, radiothérapie, chimiothérapie, immunothérapie, ou principe actif, dans le temps par mesure du ratio, par ml d'échantillon de fluide biologique, du nombre de copies d'ADN libre de toutes cellules sur le nombre de copies du premier fragment d'ADN exogène de 50-200 paires de bases, de préférence 60-160 paires de bases, de préférence encore 70-150 paires de bases et mieux de 80-140 paires de bases (ICE).

## Patentansprüche

1. Verfahren zum Nachweis und/oder zur Quantifizierung zellfreier DNA in einer Probe eines biologischen Fluids eines Probanden von Interesse, der sich von einem normalen oder gesunden Probanden unterscheidet, umfassend mindestens:
(i) einen Schritt des Extrahierens zellfreier DNA in einer Probe eines biologischen Fluids, der mindestens eine wirksame Menge eines ersten exogenen DNA-Fragments von 50-200 Basenpaaren, vorzugsweise von 60-160 Basenpaaren, weiter vorzugsweise von 70-150 Basenpaaren und besser von 80-140 Basenpaaren (ICE) zugesetzt wurde;
(ii) einen Schritt der Co-Amplifikation und Quantifizierung der zellfreien DNA und des exogenen DNA-Fragments ICE, das in Schritt (i) extrahiert wurde, und
(iii) einen Schritt der Standardisierung der Menge extrahierter zellfreier DNA, umfassend die Berechnung eines ersten Verhältnisses, je ml biologische Fluidprobe, der Anzahl der Kopien zellfreier DNA zur Anzahl der Kopien des ersten exogenen DNA-Fragments von 50-200 Basenpaaren, vorzugsweise von 60-160 Basenpaaren, weiter vorzugsweise von 70-150 Basenpaaren und besser von 80-140 Basenpaaren (ICE),
**dadurch gekennzeichnet, dass** der Proband von Interesse an Krebs leidet oder einen Krebs entwickelt oder an einem pathologischen Zustand leidet oder diesen entwickelt, der aus einem Schlaganfall oder einem Myokardinfarkt, einem akuten Nierenversagen, einer Leberzytolyse, einem Trauma, einer Transplantatabstoßung oder einer medizinischen Behandlung wie Biopsie, Operation, Strahlentherapie, Chemotherapie, Immuntherapie oder einem Wirkstoff ausgewählt ist.

2. Verfahren zum Nachweis und/oder zur Quantifizierung zellfreier DNA in einer Probe eines biologischen Fluids nach Anspruch 1, **dadurch gekennzeichnet, dass** der Proband von Interesse, der sich von einem normalen oder gesunden Probanden unterscheidet, an Krebs leidet oder diesen entwickelt.

3. Verfahren nach Anspruch 1 oder 2, **dadurch gekennzeichnet, dass** das biologische Fluid aus der Gruppe ausgewählt ist, die aus Vollblut, Serum, Plasma, Urin, Speichel, Knochenmarkausfluss, Lymphe, Liquor, Tränenflüssigkeit, Schweiß, Milch, Humor aqueus, Synovialflüssigkeit, Pleuraflüssigkeit, Peritonealflüssigkeit, Fruchtwasser, Galle, Samenflüssigkeit, Sputum, vorzugsweise Plasma, besteht.

4. Verfahren zum Nachweis und/oder zur Quantifizierung zellfreier DNA in einer Probe eines biologischen Fluids nach einem der Ansprüche 1 bis 3, **dadurch gekennzeichnet, dass** der Extraktionsschritt (i) die folgenden Schritte umfasst:
(i1) Proteolyse der Probe eines biologischen Fluids eines Probanden von Interesse, der sich von einem normalen oder gesunden Probanden unterscheidet, der mindestens ein erstes exogenes DNA-Fragment von 50-200 Basenpaaren, vorzugsweise von 60-160 Basenpaaren, weiter vorzugsweise von 70-150 Basenpaaren und besser von 80-140 Basenpaaren (ICE) zugesetzt wurde,
(i2) Isolierung der zellfreien DNA, insbesondere durch Affinität an einem Membranträger, vorzugsweise einem Siliziumdioxid-Membranträger in einer Extraktionssäule,
(i3) Elution der in Schritt (i2) isolierten zellfreien DNA in einer wässrigen Phase,
(i4) gegebenenfalls Ausfällen der zellfreien DNA,
(i5) vorzugsweise Konzentration der zellfreien DNA,
(i6) gegebenenfalls Konservierung und/oder Lagerung der zellfreien DNA.

5. Verfahren zum Nachweis und/oder zur Quantifizierung zellfreier DNA in einer Probe eines biologischen Fluids eines Probanden von Interesse, der sich von einem normalen oder gesunden Probanden unterscheidet, nach einem der Ansprüche 1 bis 4, **dadurch gekennzeichnet, dass** es eine Co-Amplifikation, insbesondere in demselben Well, einer zellfreien DNA, eines exogenen DNA-Fragments (ECD) und einer genomischen DNA-Sequenz umfasst, die aus einer mutierten DNA-Sequenz oder einer ,Donor`-DNA-Sequenz im Sonderfall eines transplantierten Probanden von Interesse, bei dem es sich um eine Abstoßung des Transplantats handeln könnte, ausgewählt ist.

6. Verfahren zum Nachweis und/oder zur Quantifizierung zellfreier DNA in einer Probe eines biologischen Fluids eines Probanden von Interesse, der sich von einem normalen oder gesunden Probanden unterscheidet, nach einem der Ansprüche 1 bis 5, **dadurch gekennzeichnet, dass** der Schritt (ii) der Amplifikation und Quantifizierung der zellfreien DNA, die in vorhergehendem Schritt extrahiert wurde, durch eine digitale PCR-, Q-PCR- oder NGS-Methode, insbesondere die digitale PCR, durchgeführt wird.

7. Verwendung des Verfahren zum Nachweis und/oder zur Quantifizierung zellfreier DNA in einer Probe eines biologischen Fluids eines Probanden von Interesse, der sich von einem normalen oder gesunden Probanden unterscheidet, nach einem der Ansprüche 1 bis 6 in einer Methode zur Analyse einer biologischen Probe eines Probanden von Interesse, der sich von einem normalen oder gesunden Probanden unterscheidet, der zellfreie DNA freisetzen kann, wobei der Proband von Interesse an Krebs leidet oder einen Krebs entwickelt oder an einem pathologischen klinischen Zustand leidet oder diesen entwickelt, der insbesondere aus einem Schlaganfall oder einem Myokardinfarkt, einem akuten Nierenversagen, einer Leberzytolyse, einem Trauma, einer Transplantatabstoßung ausgewählt ist, oder einem Probanden, der einer medizinischen Behandlung wie Biopsie, Operation, Strahlentherapie, Chemotherapie, Immuntherapie oder einem Wirkstoff unterzogen wird.

8. In-vitro-Analyseverfahren, insbesondere zur Diagnose, Prognose, Theranostik oder zur Überwachung der Entwicklung eines spezifischen physiologischen Zustands eines Probanden von Interesse, der sich von einem normalen oder gesunden Probanden unterscheidet, der zirkulierende DNA freisetzen kann, wobei der Proband von Interesse an Krebs leidet oder einen Krebs entwickelt oder an einem pathologischen klinischen Zustand leidet oder diesen entwickelt, der insbesondere aus einem Schlaganfall oder einem Myokardinfarkt, einem akuten Nierenversagen, einer Leberzytolyse, einem Trauma, einer Transplantatabstoßung ausgewählt ist, oder einem Probanden, der einer medizinischen Behandlung wie Biopsie, Operation, Strahlentherapie, Chemotherapie, Immuntherapie oder einem Wirkstoff unterzogen wird, umfassend die folgenden Schritte:
(i) zu einem Zeitpunkt t0, Nachweis und/oder Quantifizierung zellfreier DNA in einer Probe eines biologischen Fluids eines Probanden von Interesse, der sich von einem normalen oder gesunden Probanden unterscheidet, nach einem der Ansprüche 1 bis 6;
(ii) zu einem Zeitpunkt t1, Reproduktion von Schritt (i), und
(iii) Vergleich des Verhältnisses, je ml biologische Fluidprobe, der Anzahl der Kopien zirkulierender DNA zur Anzahl der Kopien des exogenen DNA-Fragments von 50-200 Basenpaaren, vorzugsweise von 60-160 Basenpaaren, weiter vorzugsweise von 70-150 Basenpaaren und besser von 80-140 Basenpaaren (ICE), erhalten zum Zeitpunkt t1 in Bezug zu demselben Verhältnis, das zum Zeitpunkt t0 erhalten wurde.

9. In-vitro-Analyseverfahren nach Anspruch 8, das insbesondere bestimmt ist zur:
- Analyse der Fluktuationen der zellfreien DNA, um das Ansprechen und/oder die Resistenz gegenüber einer medizinischen Behandlung wie Biopsie, Operation, Strahlentherapie, Chemotherapie, Immuntherapie oder Wirkstoff zu verfolgen, oder
- Analyse der Fluktuationen der zellfreien DNA, um das Auftreten einer Transplantatabstoßung zu verfolgen, oder
- Analyse der Fluktuationen der zellfreien DNA, um zu entscheiden, ob eine anschließende Analyse auf Genomanomalien sinnvoll ist, oder
- Verfolgung der Entwicklung der Konzentration von mutierter DNA, oder
- Verfolgung der Entwicklung der Konzentration von 'Donor'-DNA im speziellen Fall eines transplantierten Probanden von Interesse, der eine Transplantatabstoßung haben könnte.

10. In-vitro-Verfahren zur Diagnose, Prognose, Theranostik eines Probanden von Interesse, der sich von einem normalen oder gesunden Probanden unterscheidet, der zirkulierende DNA freisetzen kann, wobei der Proband von Interesse an Krebs leidet oder einen Krebs entwickelt oder an einem pathologischen klinischen Zustand leidet oder diesen entwickelt, der aus einem Schlaganfall oder einem Myokardinfarkt, einem akuten Nierenversagen, einer Leberzytolyse, einem Trauma, einer Transplantatabstoßung ausgewählt ist, oder einem Probanden, der einer medizinischen Behandlung wie Biopsie, Operation, Strahlentherapie, Chemotherapie, Immuntherapie oder einem Wirkstoff unterzogen wird, umfassend die folgenden Schritte:
(i) zu einem Zeitpunkt t, Nachweis und/oder Quantifizierung zellfreier DNA in einer Probe eines biologischen Fluids eines Probanden von Interesse, der sich von einem normalen oder gesunden Probanden unterscheidet, nach einem der Ansprüche 1 bis 6;
(ii) Vergleich des Verhältnisses, je ml biologische Fluidprobe, der Anzahl der Kopien zirkulierender DNA zur Anzahl der Kopien des exogenen DNA-Fragments von 50-200 Basenpaaren, vorzugsweise von 60-160 Basenpaaren, weiter vorzugsweise von 70-150 Basenpaaren und besser von 80-140 Basenpaaren (ICE), erhalten zum Zeitpunkt t in Bezug auf dasselbe Verhältnis, das bei einem Referenzprobanden (gesunder Proband, Kontrolle) erhalten wurde.

11. Verwendung, bei einem Verfahren zum Nachweis und/oder zur Quantifizierung zellfreier DNA in einer Probe eines biologischen Fluids eines Probanden von Interesse, der sich von einem normalen oder gesunden Probanden unterscheidet, nach einem der Ansprüche 1 bis 6 oder bei einem In-vitro-Analyseverfahren zur Diagnose, Prognose, Theranostik eines Probanden von Interesse, der sich von einem normalen oder gesunden Probanden unterscheidet, nach Anspruch 10, eines Kits oder Sets zur Extraktion zellfreier DNA in einer Probe eines biologischen Fluids eines Probanden von Interesse, der sich von einem normalen oder gesunden Probanden unterscheidet, wobei der Proband von Interesse an Krebs leidet oder einen Krebs entwickelt oder an einem pathologischen klinischen Zustand leidet oder diesen entwickelt, der aus einem Schlaganfall oder einem Myokardinfarkt, einem akuten Nierenversagen, einer Leberzytolyse, einem Trauma, einer Transplantatabstoßung ausgewählt ist, oder einem Probanden, der einer medizinischen Behandlung wie Biopsie, Operation, Strahlentherapie, Chemotherapie, Immuntherapie oder einem Wirkstoff unterzogen wird, umfassend:
(i) mindestens ein erstes exogenes DNA-Fragment von 50-200 Basenpaaren, vorzugsweise von 60-160 Basenpaaren, weiter vorzugsweise von 70-150 Basenpaaren und besser von 80-140 Basenpaaren (ICE);
(ii) Puffer und Enzyme, insbesondere einen Proteolysepuffer, ein Proteolyseenzym, einen Fixierpuffer, einen Lavagepuffer und einen Puffer für die Elution zellfreier DNA,
(iii) Vorrichtungen, die die Isolierung der zellfreien DNA gestatten, insbesondere Extraktionssäulen mit Siliziumdioxid-Membran und in vorteilhafter Weise Extraktionssäulen, auf denen Beladungshilfen, vorzugsweise Trichter, angebracht sind,
(iv) Multiwell-Platten, insbesondere 24-Well-DeepWell-Platten zu je 25 mL,
(v) Schraubverschlussröhrchen, vorzugsweise zu je 1,5 ml, und
(vi) eine Gebrauchsanweisung,
wobei das exogene DNA-Fragment ICE in dem Kit bereitgestellt oder separat zu dem Kit geliefert wird und ein Extraktionsset bildet.

12. Verwendung nach Anspruch 11 in einem In-vitro-Verfahren nach einem der Ansprüche 1 bis 10 eines Kits oder Sets zur Extraktion zellfreier DNA nach Anspruch 11 in Verbindung mit einem Kit zur Amplifikation und Quantifizierung zellfreier DNA, die zusammen oder getrennt bereitgestellt werden, wobei das Kit zur Amplifikation und Quantifizierung zellfreier DNA mindestens umfasst:
(i) Sense- und Antisense-Primer, die jeweils spezifisch für die zellfreie DNA des Probanden von Interesse sind, der sich von einem normalen oder gesunden Probanden unterscheidet, und das exogene DNA-Fragment nach Anspruch 1,
(ii) Sonden, die jeweils für die zellfreie DNA des Probanden von Interesse, der sich von einem normalen oder gesunden Probanden unterscheidet, und für das exogene DNA-Fragment nach Anspruch 1 markiert sind,
(iii) Reaktionspuffer,
(iv) Multiwell-Platten und
(v) eine Gebrauchsanweisung.

13. Verwendung, bei einem Verfahren zum Nachweis und/oder zur Quantifizierung zellfreier DNA in einer Probe eines biologischen Fluids eines Probanden von Interesse, der sich von einem normalen oder gesunden Probanden unterscheidet, nach einem der Ansprüche 1 bis 6 oder bei einem In-vitro-Analyseverfahren nach Anspruch 8 oder bei einem In-vitro-Verfahren zur Diagnose, Prognose, Theranostik eines Probanden von Interesse, der sich von einem normalen oder gesunden Probanden unterscheidet, nach Anspruch 10, mindestens eines ersten exogenen DNA-Fragments von 50-200 Basenpaaren, vorzugsweise von 60-160 Basenpaaren, weiter vorzugsweise von 70-150 Basenpaaren und besser von 80-140 Basenpaaren (ICE), das es gestattet, eine Methode zur Extraktion und Quantifizierung zellfreier DNA zu standardisieren und/oder die Fluktuationen zellfreier DNA bei einem Probanden von Interesse, der sich von einem normalen oder gesunden Probanden unterscheidet, der zellfreie DNA freisetzen kann, zu analysieren, wobei der Proband von Interesse an Krebs leidet oder einen Krebs entwickelt oder an einem pathologischen klinischen Zustand leidet oder diesen entwickelt, der insbesondere aus einem Schlaganfall oder einem Myokardinfarkt, einem akuten Nierenversagen, einer Leberzytolyse, einem Trauma, einer Transplantatabstoßung ausgewählt ist oder einem Probanden, der einer medizinischen Behandlung wie Biopsie, Operation, Strahlentherapie, Chemotherapie, Immuntherapie oder einem Wirkstoff unterzogen wird, im Laufe der Zeit durch Messen des Verhältnisses, je ml biologische Fluidprobe, der Anzahl der Kopien freier DNA zur Anzahl der Kopien des ersten exogenen DNA-Fragments von 50-200 Basenpaaren, vorzugsweise von 60-160 Basenpaaren, weiter vorzugsweise von 70-150 Basenpaaren und besser von 80-140 Basenpaaren (ICE).

## Claims

1. A process for detecting and/or quantifying cell-free DNA from a biological fluid sample from a subject of interest, who is not a normal or healthy subject, comprising at least:
(i) a step of extracting cell-free DNA from a biological fluid sample in which has been added at least an effective amount of a first exogenous DNA fragment of 50-200 base pairs, preferably 60-160 base pairs, more preferably 70-150 base pairs and better 80-140 base pairs (ICE);
(ii) a step of co-amplifying and quantifying the cell-free DNA and the exogenous ICE DNA fragment extracted in step (i), and
(iii) a step of standardising the amount of extracted cell-free DNA comprising calculating a first ratio, per ml of biological fluid sample, of the copy number of cell-free DNA to the copy number of the first exogenous DNA fragment of 50-200 base pairs, preferably 60-160 base pairs, more preferably 70-150 base pairs and better 80-140 base pairs (ICE),
**characterised in that** the subject of interest is affected by or likely to develop a cancer or a pathological condition selected from stroke or myocardial infarction, acute renal failure, hepatic cytolysis, trauma, graft rejection or a medical treatment such as biopsy, surgery, radiotherapy, chemotherapy, immunotherapy or active principle.

2. The process for detecting and/or quantifying cell-free DNA from a biological fluid sample according to claim 1, **characterised in that** the subject of interest, who is not a normal or healthy subject, is affected by or likely to develop a cancer.

3. The process according to claim 1 or 2, **characterised in that** the biological fluid is selected from the group consisting of whole blood, serum, plasma, urine, saliva, bone marrow effluent, lymph, cerebrospinal fluid, lacrimal fluid, sweat, milk, aqueous humour, synovial fluid, pleural fluid, peritoneal fluid, amniotic fluid, bile, seminal fluid, expectorations, preferably plasma.

4. The process for detecting and/or quantifying cell-free DNA from a biological fluid sample according to one of claims 1 to 3, **characterised in that** the extraction step (i) comprises the following steps:
(i1) Proteolysis of said biological fluid sample of a subject of interest, who is not a normal or healthy subject, to which has been added at least a first exogenous DNA fragment of 50-200 base pairs, preferably 60-160 base pairs, more preferably 70-150 base pairs and better 80-140 base pairs (ICE),
(i2) Isolation of cell-free DNA, in particular by affinity on a membrane support, preferably a silica membrane support in an extraction column,
(i3) Elution of the cell-free DNA isolated in step (i2) in an aqueous phase,
(i4) Optionally precipitation of the cell-free DNA,
(i5) Advantageously concentration of the cell-free DNA,
(i6) Optionally preservation and/or storage of said cell-free DNA.

5. The process for detecting and/or quantifying cell-free DNA from a biological fluid sample from a subject of interest, who is not a normal or healthy subject, according to any one of claims 1 to 4, **characterised in that** it comprises co-amplifying a cell-free DNA, an exogenous DNA fragment (ICE) and a genomic DNA sequence selected from a mutated DNA sequence or a donor DNA sequence in the particular case of a transplanted subject of interest who may have a graft rejection.

6. A process for detecting and/or quantifying cell-free DNA from a biological fluid sample from a subject of interest, who is not a normal or healthy subject, according to any one of the claims 1 to 5, **characterised in that** the step (ii) of amplification and quantification of the cell-free DNA extracted in the preceding step is carried out by a digital PCR, Q-PCR and/or NGS process, in particular digital PCR.

7. Use of the process for detecting and/or quantifying cell-free DNA from a biological fluid sample from a subject of interest, who is not a normal or healthy subject, according to any one of claims 1 to 6, in a method for analysing a biological sample from a subject of interest, who is not a normal or healthy subject and who is likely to release cell-free DNA, said subject of interest being affected by or likely to develop a cancer or a pathological clinical condition selected in particular from stroke or myocardial infarction, acute renal failure, hepatic cytolysis, trauma, graft rejection, or a subject undergoing a medical treatment such as biopsy, surgery, radiotherapy, chemotherapy, immunotherapy, or active principle.

8. An *in vitro* analysis process, in particular for diagnosis, prognosis, theranosis, or the monitoring of changes in a specific physiological state of a subject of interest, who is not a normal or healthy subject and who is likely to release circulating DNA, said subject of interest being affected or developing a cancer or a clinical condition selected in particular from stroke or myocardial infarction, acute renal failure, hepatic cytolysis, trauma, graft rejection, or a subject undergoing a medical treatment such as biopsy, surgery, radiotherapy, chemotherapy, immunotherapy, or active principle, comprising the following steps:
(i) at a time point t0, detecting and/or quantifying cell-free DNA from a biological fluid sample from a subject of interest, who is not a normal or healthy subject, according to any one of claims 1 to 6;
(ii) at a time point t1, repeating step (i), and
(iii) comparing the ratio, per ml of biological fluid sample, of the copy number of circulating DNA to the copy number of exogenous DNA fragment of 50-200 base pairs, preferably 60-160 base pairs, more preferably 70-150 base pairs and better 80-140 base pairs (ICE), obtained at time point t1, with the same ratio obtained at time point t0.

9. The *in vitro* process according to claim 8, intended in particular to:
- analyse fluctuations in cell-free DNA to monitor response and/or resistance to medical treatment such as biopsy, surgery, radiotherapy, chemotherapy, immunotherapy, or active principle, or
- analyse fluctuations in cell-free DNA to monitor the occurrence of graft rejection, or
- analyse fluctuations in cell-free DNA to decide whether to conduct a subsequent analysis of genomic abnormalities, or
- monitor changes in the concentration of mutated DNA, or
- monitor changes in the concentration of donor DNA in the particular case of a transplanted subject of interest likely to have a graft rejection.

10. *An in vitro* process for diagnosis, prognosis, theranosis of a subject of interest, who is not a normal or healthy subject and who is likely to release circulating DNA, said subject of interest being affected or developing a cancer or a pathological clinical condition selected in particular from stroke or myocardial infarction, acute renal failure, hepatic cytolysis, trauma, graft rejection, or a subject undergoing a medical treatment such as biopsy, surgery, radiotherapy, chemotherapy, immunotherapy, or active principle, comprising the following steps:
(i) at a time point t, detecting and/or quantifying cell-free DNA from a biological fluid sample from a subject of interest, who is not a normal or healthy subject, according to any one of claims 1 to 6;
(ii) comparing the ratio, per ml of biological fluid sample, of the copy number of circulating DNA to the copy number of exogenous DNA fragment of 50-200 base pairs, preferably 60-160 base pairs, more preferably 70-150 base pairs and better 80-140 base pairs (ICE), obtained at time point t, with the same ratio obtained in a reference subject (healthy subject, control)

11. Use, in a method for detecting and/or quantifying cell-free DNA from a biological fluid sample from a subject of interest, who is not a normal or healthy subject, according to anyone of claims 1 to 6, or in an *in vitro* analysis process according to claim 8, or in an *in vitro* process for diagnosis, prognosis, theranosis of a subject of interest, who is not a normal or healthy subject according to claim 10, of a kit or set for extraction of cell-free DNA from a biological fluid sample of a subject of interest, who is not a normal or healthy subject, said subject of interest being affected by or developing a cancer or a pathological clinical condition selected from stroke or myocardial infarction, acute renal failure, hepatic cytolysis, trauma, graft rejection, or a subject undergoing a medical treatment such as biopsy, surgery, radiotherapy, chemotherapy, immunotherapy, or active principle, comprising:
(i) At least a first exogenous DNA fragment of 50-200 base pairs, preferably 60-160 base pairs, more preferably 70-150 base pairs and better 80-140 base pairs (ICE);
(ii) Buffers and enzymes, in particular a cell-free DNA proteolysis buffer, proteolysis enzyme, binding buffer, wash buffer, and elution buffer,
(iii) Devices for isolating cell-free DNA, in particular silica membrane extraction columns and advantageously extraction columns surmounted by loading accessories, preferably funnels,
(iv) Multi-well plates, in particular 24-well DeepWell 25 ml plates
(v) Screw-cap tubes, preferably 1.5 ml, and
(vi) A user manual,
said exogenous ICE DNA fragment being provided in said kit or provided separately to said kit, forming an extraction set.

12. The use according to claim 11 in an *in vitro* process according to any one of claims 1 to 10, of a kit or set for extraction of cell-free DNA as defined in claim 11, in combination with a cell-free DNA amplification and quantification kit, provided together or separately, said cell-free DNA amplification and quantification kit comprising at least:
(i) specific sense and antisense primers for the cell-free DNA of the subject of interest, who is not a normal or healthy subject, and for the exogenous DNA fragment as defined in claim 1,
(ii) probes marked respectively for cell-free DNA of the
subject of interest, who is not a normal or healthy subject, and for exogenous DNA fragment as defined in claim 1,
(iii) reaction buffers
(iv) multi-well plates and
(v) a user manual.

13. Use, in a method for detecting and/or quantifying cell-free DNA from a biological fluid sample of a subject of interest, who is not a normal or healthy subject, according to any one of claims 1 to 6, or in an in vitro analysis process according to claim 8, or in an in vitro process for diagnosis, prognosis , theranosis of a subject of interest, who is not a normal or healthy subject, according to claim 10, of at least a first exogenous DNA fragment of 50-200 base pairs, preferably 60-160 base pairs, more preferably 70-150 base pairs and better 80-140 base pairs (ICE), making it possible to standardise a process for extracting and quantifying cell-free DNA and/or analysing the fluctuations in cell-free DNA in a subject of interest, who is not a normal or healthy subject, said subject of interest being affected by or developing a cancer or a pathological clinical condition, selected from stroke or myocardial infarction, acute renal failure, hepatic cytolysis, trauma, graft rejection, or a subject undergoing a medical treatment such as biopsy, surgery, radiotherapy, chemotherapy, immunotherapy, or active principle, over time by measuring the ratio, per ml of biological fluid sample, of the copy number of cell-free DNA to the copy number of the first exogenous DNA fragment of 50-200 base pairs, preferably 60-160 base pairs, more preferably 70-150 base pairs and better 80-140 base pairs (ICE).
